**Europäisches Patentamt**

**European Patent Office**

**Office européen des brevets**

(19)

(11) **EP 1 624 074 A1**

(12) **DEMANDE DE BREVET EUROPEEN**

(43) Date de publication:
**08.02.2006 Bulletin 2006/06**

(51) Int Cl.:
***C12Q 1/68*** (2006.01)

(21) Numéro de dépôt: **04292013.2**

(22) Date de dépôt: **06.08.2004**

(84) Etats contractants désignés:
**AT BE BG CH CY CZ DE DK EE ES FI FR GB GR HU IE IT LI LU MC NL PL PT RO SE SI SK TR**
Etats d'extension désignés:
**AL HR LT LV MK**

(71) Demandeur: **Neurolab**
**75015 Paris (FR)**

(72) Inventeurs:
- **Leporrier, Nathalie**
**14000 Caen (FR)**
- **Herrou, Michel**
**14210 Gravus (FR)**
- **Feldblum, Sophie**
**75015 Paris (FR)**

- **Simonin, Pierre-Yves**
**76600 Le Havre (FR)**
- **Cornuel, Jean-François**
**75014 Paris (FR)**

(74) Mandataire: **Vialle-Presles, Marie José et al**
**Cabinet ORES,**
**36,rue de St Pétersbourg**
**75008 Paris (FR)**

Remarques:
Le listage des séquences, qui est publié en annexe aux documents de la demande, a été produit après la date de dépôt. Le demandeur a déclaré qu'il ne contient pas d'élément s'étendant au-delà du contenu de la demande telle qu'elle a été déposée.

(54) **Marqueurs et procédés pour le dépistage prénatal d'anomalies chromosomiques**

(57) Dépistage prénatal d'anomalies chromosomiques, plus particulièrement, par l'identification de nouveaux marqueurs d'anomalies chromosomiques, notamment de la trisomie 21, dont le dosage à partir d'un échantillon de fluide biologique de la femme enceinte permet, seul ou en en combinaison avec d'autres dosages, de déterminer la probabilité que le foetus soit atteint d'une anomalie chromosomique, avec une précision supérieure à celle des tests actuellement utilisés.

EP 1 624 074 A1

**Description**

La présente invention concerne le dépistage prénatal d'anomalies chromosomiques. Plus particulièrement, l'invention est basée sur l'identification de nouveaux marqueurs d'anomalies chromosomiques, notamment de la trisomie 21, dont le dosage à partir d'un échantillon de fluide biologique de la femme enceinte permet, seul ou en en combinaison avec d'autres dosages, de déterminer la probabilité que le foetus soit atteint d'une anomalie chromosomique, avec une précision supérieure à celle des tests actuellement utilisés.

La trisomie 21 ou Syndrome de Down est la plus fréquente des anomalies chromosomiques viables. A l'origine de handicaps psychomoteurs, elle affecte une naissance sur 800, et sachant qu'environ 800 000 naissances sont observées en France, l'incidence est de 1000 enfants trisomiques par an.

Son phénotype caractéristique permet le diagnostic clinique à la naissance. Environ 40% des enfants atteints de trisomie 21 sont également porteurs de malformations, dont les plus fréquentes sont des cardiopathies congénitales. Leur espérance de vie est maintenant de plus de 50 ans.

Le risque de trisomie augmente avec l'âge maternel, surtout après 35 ans (croissance exponentielle). Le risque est également augmenté si l'un des parents est porteur d'une translocation équilibrée ou si le couple a déjà donné naissance à un enfant avec anomalie chromosomique. Les anomalies foetales dépistées à l'échographie peuvent également conduire à la prescription d'un caryotype foetal.

Le dépistage anténatal de la trisomie 21 consiste en l'étude du caryotype foetal, soit sur cellules amniotiques, soit sur cellules trophoblastiques. Le prélèvement est à risque de fausse couche. Le caryotype est un examen long qui se pratique en laboratoire spécialisé et autorisé par le Ministère de la Santé. Il est donc réservé à une population de femmes dites "à risque élevé d'anomalies chromosomiques".

La proportion de cas de trisomie 21 dépistés sur des critères d'âge maternel et de signes d'appel échographiques est actuellement d'environ un tiers. Cependant la prévalence de la trisomie 21 à la naissance n'a pas significativement diminuée en raison de l'augmentation régulière de l'âge moyen des femmes enceintes.

Depuis 1980, la prise en charge du caryotype foetal par l'Assurance Maladie est fixée par voie réglementaire (Arrêté du 29 octobre 1991 paru au Journal officiel du 16 novembre 1991). Le caryotype foetal est pris en charge financièrement en présence de l'une des indications suivantes :

1. Âge de la femme égal ou supérieur à 38 ans à la date du prélèvement
2. Anomalie chromosomique parentale
3. Antécédent, pour le couple, de grossesse(s) avec caryotype anormal
4. Diagnostic du sexe pour les maladies liées au sexe
5. Signes d'appel échographiques suivants : anomalies morphologiques du foetus démontrées, internes ou externes, retard de croissance intra-utérin avéré, anomalies de quantité de liquide amniotique.

Depuis quelques années, de nombreuses équipes cherchent donc à identifier un ou des marqueurs sériques mesurables dans le sang maternel et qui pourraient permettre une évaluation du risque sur une population plus large.

En 1984, un premier marqueur sérique maternel de risque, l'alpha-foetoprotéine (AFP), a été identifié : le taux d'AFP, en moyenne plus bas dans les grossesses avec trisomie 21, peut être utilisé pour calculer le risque de trisomie. En 1987, un autre marqueur, l'hormone gonadotrophine chorionique (hCG) s'est révélé plus élevé dans les grossesses avec trisomie 21. Depuis, beaucoup d'autres marqueurs sériques de risque de trisomie 21 ont été identifiés.

Les marqueurs les plus étudiés ont été : l'hCG, l'AFP, l'oestriol non conjugué (uE3), la sous-unité β de l'hCG. Chaque marqueur utilisé individuellement est moins performant que son association à un ou plusieurs autres.

En France, une étude pilote nationale multicentrique et plusieurs études régionales ont été effectuées. La demande des femmes s'est progressivement affirmée et, en 1994, plus de 10% des femmes enceintes ont eu un dosage de marqueurs sériques.

Ainsi, en France, depuis 1997, les marqueurs sériques maternels (hCG, uE3 et AFP) sont dosés et pris en charge par la Sécurité Sociale. Le test est côté B145 soit 39 euros 15. Un prélèvement de sang entre la 15ème et la 18ème semaine d'aménorrhée, pour le dosage d'au moins deux de ces marqueurs, est effectué, permettant un calcul de risque tenant compte du risque *a priori* de l'âge de la mère modulé selon les valeurs observées de ces marqueurs. Le risque seuil considéré comme décisionnel pour proposer un dépistage prénatal (amniocentèse) est arbitrairement fixé à 1/250.

Le dosage des marqueurs sériques n'établit pas à lui seul le diagnostic de trisomie 21. C'est un test de dépistage qui permet d'estimer le risque d'attendre un enfant trisomique 21.

L'évaluation du risque de trisomie 21 par dosage des marqueurs sériques chez les femmes qui le souhaitent est largement effectuée aujourd'hui. En 1998 une étude menée par l'INSERM permettait d'estimer le nombre de dosages à 572000 soit 76 % des naissances (certainement 85 % en 2004).

Cette politique est également appliquée dans les autres pays européens et anglo-saxons où la fréquence de la trisomie 21 est la même.

La sensibilité du dépistage est d'environ 60 % si le seuil de risque choisi pour accéder à l'amniocentèse est de 1/250. Cela conduit à proposer une amniocentèse à environ 5 % des femmes enceintes. La valeur prédictive positive (spécificité) est de l'ordre de 1 à 2 %. La sensibilité du dépistage est meilleure, à taux d'amniocentèse égal, si les marqueurs sont plus discriminants et combinés entre eux. Les combinaisons les plus utilisées actuellement sont le triple test (hCG + AFP + uE3) et les doubles tests, hCG + AFP ou hCG +uE3, ce dernier étant le meilleur.

Les faux positifs du dépistage sont les amniocentèses à caryotype normal.. Le nombre de faux positifs doit être réduit le plus possible du fait du risque de perte foetale après amniocentèse (environ 1 %). Par ailleurs, ils peuvent générer une grande anxiété chez les femmes dans l'attente des résultats du caryotype.

Les faux négatifs du dépistage sont les cas de trisomie 21 non dépistés. Ils aggravent le choc émotionnel au moment de la naissance. En effet, les couples ayant bénéficié d'un dosage de marqueurs sériques comprennent mal la notion de risque résiduel et pensent souvent que le test est un moyen diagnostique. L'échec du dépistage est alors durement ressenti.

Cette situation n'est pas satisfaisante dans la mesure où elle laisse passer 30 à 40 % des grossesses avec trisomie 21. Il faut trouver une technique visant à augmenter la sensibilité et la spécificité, réduire le nombre de procédures inutilement invasives (faux positifs) et surtout réduire le nombre de faux négatifs (30 à 40 %).

D'autres procédures sont en cours d'évaluation et non remboursées par la Sécurité Sociale, notamment les marqueurs sériques du premier trimestre de la grossesse. Il s'agit de la protéine PAPP-A *(pregnancy* associated *plasma protein* A), de la sous-unité bêta libre de hCG et de la mesure de la clarté nucale à 12 semaines d'aménorrhée. Certaines équipes essayent également de mettre au point la mise en évidence de cellules foetales ou de l'ADN foetal libre dans le sang maternel sans parvenir aujourd'hui à un dépistage efficace.

Les performances des marqueurs sériques dépendent de la stratégie choisie : seuil de risque au-delà duquel une amniocentèse est proposée, nature des marqueurs utilisés, et type d'associations entre eux. Certaines combinaisons de marqueurs sont plus performantes que d'autres (Wald et al, Lancet, 2003, 8, 361, 855-6).

Une étude récente portant sur 120 000 grossesses a montré que les foetus trisomiques 21 détectés en prénatal par les marqueurs sériques ou anomalies échographiques (clarté nucale) auraient avorté spontanément plus que les foetus trisomiques non détectables par ces méthodes (Leporrier N. et al., BJOG, 2003, 110, 1, 18-21). Ceci diminue donc l'efficacité de ce dépistage et incite à rechercher d'autres bio-marqueurs plus performants.

Dans une démarche originale visant à découvrir de nouveaux bio-marqueurs plus spécifiques de la trisomie 21, et/ou à déterminer un profil d'expression particulier, basé sur plusieurs molécules, les inventeurs ont conduit des études à partir de 280 sérums congelés de femmes enceintes avec foetus trisomiques 21 (incluant celles qui ont été détectées et celles qui n'ont pas été détectées par les marqueurs sériques du second trimestre), et plus de 280 sérums témoins de femmes sans foetus trisomiques 21 dont les issues sont connues.

Le placenta est une barrière entre la mère et le foetus, mais il est prouvé que certaines molécules de petite masse moléculaire passent cette barrière. Les inventeurs ont donc choisi d'appliquer la technique protéomique dans le sérum des femmes enceintes, afin de déterminer un profil d'expression différentielle permettant de distinguer de façon quasi certaine celles avec foetus trisomiques 21 par comparaison avec des sérums de femmes enceintes normales. Cette démarche, illustrée dans la partie expérimentale ci-après, leur a permis d'identifier plusieurs marqueurs permettant de distinguer de façon quasi certaine les sérums de femmes avec foetus trisomiques 21 par comparaison avec des sérums de femmes enceintes normales.

La présente invention porte donc, en premier lieu, sur l'utilisation d'un ou plusieurs marqueur(s) choisi(s) dans la liste (i) ci-après. PM3061 ; PM3101 ; PM3156 ; PM3166 ; PM3181 ; PM3214 ; PM3223 ; PM3233 ; PM3313 ; PM3320 ; PM3321 ; PM3371 ; PM3394 ; PM3400 ; PM3442 ; PM3486 ; PM3504 ; PM3678 ; PM3705 ; PM3815 ; PM3846 ; PM4059 ; PM4067 ; PM4110 ; PM4111 ; PM4152 ; PM4168 ; PM4186 ; PM4197 ; PM4201 ; PM4217 ; PM4279 ; PM4286 ; PM4295 ; PM4300 ; PM4317 ; PM4332 ; PM4349 ; PM4410 ; PM4412 ; PM4463 ; PM4470 ; PM4530 ; PM4583 ; PM4650 ; PM4682 ; PM4706 ; PM4722 ; PM4816 ; PM4821 ; PM4860 ; PM4893 ; PM4935 ; PM5008 ; PM5033 ; PM5222 ; PM5625 ; PM5630 ; PM5695 ; PM5712 ; PM5720 ; PM5756 ; PM5793 ; PM5799 ; PM5982 ; PM6222 ; PM6299 ; PM6423 ; PM6430 ; PM6439 ; PM6461 ; PM6470 ; PM6482 ; PM6548 ; PM6605 ; PM6620 ; PM6624 ; PM6636 ; PM6659 ; PM6679 ; PM6764 ; PM6782 ; PM6808 ; PM6937 , PM7019 ; PM7020 ; PM7652 ; PM8112 ; PM8176 ; PM8206 ; PM8220 ; PM8226 ; PM8558 ; PM8563 ; PM8581 ; PM8594 ; PM8627 ; PM8691 ; PM8695 ; PM8717 ; PM8809 ; PM8872 ; PM8917 ; PM8929 ; PM9291 ; PM9358 ; PM9360 ; PM9401 ; PM9907 ; PM9617 ; PM10051 ; PM11093 ; PM11369 ; PM12434 ; PM12575 ; PM12862 ; PM13313 ; PM13316 ; PM14027 ; PM14035 ; PM14430 ; PM16672 ; PM16995,56 ; PM17262 ; PM17348,75 ; PM17386 ; PM17581,18 ; PM18901,94 ; PM19139,2 ; PM19558,27 ; PM19624,73 ; et PM19845,94, pour le diagnostic prénatal d'une anomalie foetale.

L'invention porte également sur un procédé de dépistage *in vitro* pour déterminer si une femme enceinte est porteuse d'un foetus atteint d'une anomalie, comprenant une étape de dosage, dans un échantillon biologique de ladite femme enceinte, d'au moins un marqueur sélectionné dans la liste (i) ci-dessus.

Au sens de la présente invention, le mot "dosage" désigne une mesure quantitative ou semi-quantitative au sens large, par exemple une quantification relative d'un composé, par rapport au même composé dans un autre échantillon, ou par rapport à un autre composé dans le même échantillon. Ainsi, dans le présent texte, une comparaison de profils, obtenus par exemple par spectrométrie de masse, est assimilé à un "dosage".

Dans une mise en oeuvre préférée du procédé de l'invention, il comprend une étape de dosage d'au moins trois marqueurs, dont l'un au moins est sélectionné dans la liste (i) ci-dessus, et les deux autres sont sélectionnés soit dans cette même liste, soit parmi les marqueurs déjà caractérisés et utilisés aujourd'hui, tels que l'un des marqueurs du second trimestre - l'hormone gonadotrophine chorionique (hCG) ou sa sous-unité β libre, l'oestriol non conjugué (uE3), l'α-foetoprotéine (AFP) et/ou l'inhibine A - ou du premier trimestre -sous-unité β libre d'hCG et/ou protéine plasmatique associée à la grossesse (PAPP-A). Alternativement, un des dosages peut être remplacé par l'observation de la clarté nucale, au premier trimestre.

Par exemple, un "triple test amélioré", basé sur le dosage de hCG, uE3, et un des marqueurs cités ci-dessus, est partie intégrante de l'invention. Alternativement, le dosage d'un marqueur de la liste (i) ci-dessus peut être combiné à un ou plusieurs autre(s) test(s) connu(s), comme par exemple l'examen de la clarté nucale.

L'invention permet de dépister des anomalies chromosomiques, en particulier la trisomie 21, la trisomie 18 et une délétion du bras long du chromosome 7, mais également d'autres anomalies congénitales, dépistées actuellement par échographie, par exemple le *Spina Bifida.* En outre, l'invention peut être appliquée au dépistage de pathologies génétiques ou infectieuses (virale, bactérienne, ou parasitaire) ou métaboliques du foetus, ou à la recherche d'un phénotype précis et en particulier du sexe du foetus.

Le procédé de l'invention implique une étape de dosage à partir d'un échantillon biologique provenant de la femme enceinte, lequel peut être constitué de n'importe quel fluide corporel, tel qu'un échantillon de sang, sérum, plasma, urines...

La présente invention est applicable au dépistage prénatal au cours du second trimestre, ou plus précocement, au cours du premier trimestre de la grossesse.

Dans l'ensemble du texte, les marqueurs identifiés par les inventeurs sont désignés par leur poids moléculaire (PM). Ce poids moléculaire est celui mesuré par spectrométrie de masse, selon les protocoles décrits ci-après dans la partie expérimentale (en conditions non dénaturantes). Ainsi, "PM3371" désigne la molécule identifiée par un pic à 3371 Da par spectrométrie de masse effectuée en utilisant une surface de séparation anionique dans les conditions résumées dans le Tableau 20 ci-après. "PM6423" désigne la molécule identifiée par un pic à 6423 Da dans des conditions différentes, en utilisant une surface de séparation hydrophobe. Le Tableau 14 présenté dans la partie expérimentale ci-dessous indique, pour chaque marqueur, s'il a été identifié en phase séparative anionique ou hydrophobique. Il est important de noter que les poids moléculaires des marqueurs identifiés par les inventeurs ont été mesurés dans des conditions précises, notamment de calibration, et avec une instrumentation donnée, en particulier avec un laser bien réglé. Dans ces conditions, la mesure des poids moléculaires des différents marqueurs est reproductible, avec un coefficient de variation très inférieur à 10 %, typiquement de l'ordre de 0,3 à 0,5 %, pour les marqueurs de poids moléculaire inférieur à 12000 Da. Du fait des calibrants utilisés, ce coefficient de variation est de l'ordre de 10 à 25 % pour les marqueurs de poids moléculaire supérieur à 12000 Da. Comme précisé dans la partie expérimentale ci-dessous (titre C-3), le choix du calibrant n'influence pas les valeurs des intensités des pics observés. L'utilisation de calibrants différents de ceux utilisés par les inventeurs peut éventuellement conduire à une variation au niveau de la masse mesurée pour le marqueur. Les pics correspondant à chacun des marqueurs restent toutefois toujours identifiables.

Parmi les marqueurs cités ci-dessus, les inventeurs ont identifié un sous-groupe de 19 marqueurs particulièrement performants, qui sont cités dans la liste (ii) ci-après : PM3371 ; PM3442 ; PM3486 ; PM4152 ; PM4168 ; PM4186 ; PM4201 ; PM4300 ; PM5695 ; PM6222 ; PM4682 ; PM5625 ; PM6423 ; PM6439 ; PM6620 ; PM6636 ; PM9360 ; PM14035 ; et PM17262. Dans une mise en oeuvre préférée de l'invention, au moins un marqueur est choisi dans la liste (ii).

Selon différentes variantes de l'invention, le nombre de marqueurs sélectionnés parmi les molécules identifiées par les inventeurs peut être égal à 1, 2, 3, 4, 5, 6, 7, 8, 9, 10, 15, 20 ou davantage, qu'il s'agisse de marqueurs sélectionnés dans le sous-groupe des 19 marqueurs potentiellement les plus pertinents (liste (ii)), ou dans la liste (i) plus étendue. L'homme du métier déterminera le nombre de marqueurs à doser en fonction de contraintes d'efficacité (recherche d'un test le plus sensible et sélectif possible), et de contraintes économiques.

Selon une de ces variantes particulières, l'invention porte sur un procédé tel que décrit plus haut, comportant une étape de dosage d'au moins trois marqueurs nouvellement identifiés par les inventeurs, sélectionnés soit dans la liste étendue (i), soit dans la liste restreinte (ii). Ce procédé peut en outre comporter une étape de dosage, dans l'échantillon biologique de la femme enceinte, d'un ou plusieurs autre(s) marqueur(s) choisi(s) parmi l'hormone gonadotrophine chorionique (hCG); la sous-unité β de l'hCG ; l'oestriol non conjugué (uE3); et l'alpha-foetoprotéine (AFP).

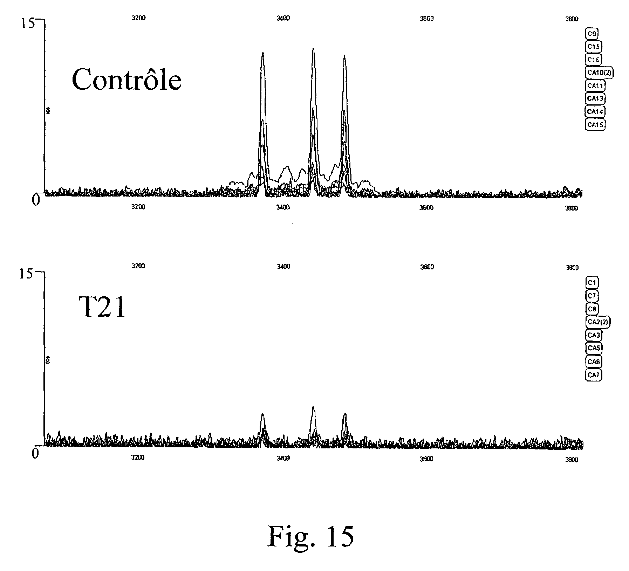

Parmi les marqueurs caractérisés par les inventeurs, PM3371, PM3442, et PM3486 ont été identifiés comme étant les défensines neutrophiles humaines HND-2, HND-1 et HND-3, respectivement (voir partie expérimentale, D-9). Ces défensines sont surexprimées ou exprimées normalement chez les contrôles, et sous-exprimées en cas de trisomie (fig. 15). Selon une mise en oeuvre préférée du procédé de l'invention, au moins un des marqueurs pour lesquels le dosage est effectué est sélectionné parmi PM3371, PM3442, et PM3486. Un taux élevé du marqueur en question est indicatif d'un foetus sain, tandis qu'un taux bas est indicatif d'une probabilité élevée de trisomie.

L'invention porte également sur l'utilisation d'une ou plusieurs défensines neutrophiles humaines choisies parmi HND-1, HND-2 et HND-3, comme marqueur(s) pour le diagnostic prénatal d'une anomalie chromosomique.

Selon une autre mise en oeuvre particulière de l'invention, au moins un des marqueurs considérés est sélectionné parmi PM6423, PM6439, PM6620, et PM6636.

Bien entendu, les connaissances de l'homme du métier lui permettent de compléter les procédés de l'invention, par exemple en tenant compte de l'âge de la femme enceinte ou de toute autre variable (semaine de gestation, ...) utilisée comme critère d'ajustement.

Dans une mise en oeuvre préférée des procédés de l'invention, ces procédés comportent également, pour chaque marqueur dosé, une étape de comparaison de la concentration mesurée de ce marqueur dans l'échantillon biologique de la femme enceinte, à des valeurs de référence de la concentration de ce marqueur chez des femmes enceintes portant des foetus normaux, et chez des femmes enceintes portant des foetus atteints d'une anomalie chromosomique et/ou génétique connue, la comparaison étant indicatrice du risque que la femme enceinte porte un foetus atteint d'une anomalie chromosomique et/ou génétique. Ces références seront choisies par l'homme du métier de façon à correspondre à l'âge gestationnel du prélèvement effectué chez la femme enceinte.

Selon une mise en oeuvre particulière de l'invention, les anomalies chromosomiques dont le dépistage est recherché comprennent au moins une maladie sélectionnée dans le groupe constitué par le syndrome de Down (trisomie 21), le syndrome d'Edwards (trisomie 18), le syndrome de Patau (trisomie 13), le syndrome de Turner, le syndrome de Klinefelter, la monosomie X, le syndrome de 1'X fragile, et le syndrome penta X. L'anomalie la plus souvent recherchée est le syndrome de Down.

Les procédés de dépistage selon l'invention peuvent également comporter une étape initiale de préparation de l'échantillon. Par exemple, cette étape peut comprendre un fractionnement en fonction de la masse des protéines, et/ou une étape de déplétion en albumine ou autres protéines de transport, et/ou une étape de purification des protéines sur une surface préparative de type anionique et/ou hydrophobe.

Dans une réalisation particulière de ces procédés, illustrée dans les exemples, le dosage d'au moins un marqueur est effectué par spectrométrie de masse, par exemple par spectrométrie de masse à basse résolution (SELDI-TOF) ou à haute résolution (Bruker).

Bien entendu, des techniques de séparation, purification, ou de dosage différentes de celles mentionnées ci-dessus peuvent être utilisées dans le cadre de l'invention. En particulier, deux types de technologies peuvent être envisagées pour développer un dispositif de diagnostic selon l'invention : la comparaison de profils, ou le dosage ponctuel des marqueurs. A titre d'exemples non limitatifs, on peut citer les techniques suivantes :

- Techniques de séparation : H.P.L.C., L.C. *(Liquid Chromatography),* C.E. *(Capillary Electrophoresis)* toutes technologies de type "microfluidique", gels bi- ou mono-dimensionnels, séparation en mode réverse *(reverse phase)* sur support solide plan ou non (*planar surface, bead surface),* les échantillons biologiques pouvant être ou non préalablement pré-fractionnés, par exemple sur membrane (Type Vivaspin de Vivascience mais aussi les spin columns de Millipore, Type Proteospin), et/ou déplétés ou non des protéines majoritaires (albumine, ovalbumine, immunoglobulines etc...), et/ou associés par affinité.

- Des anticorps peuvent être produits contre les marqueurs de l'invention, par n'importe quelle technique, y compris anticorps monoclonaux, synthèse chimique, production biologique (voie animale [exemple Bioprotein etc..], voie végétale [exemple Lemna - Biolex-Bayer, Meristems Therapeutics aussi etc...]). Ils peuvent ensuite être utilisés, soit pour effectuer directement un immuno-dosage des marqueurs correspondants, soit pour purifier les marqueurs préalablement à leur dosage.

- Dosage par comparaison de profils (spectres de masse) : outre les technologies décrites dans la partie expérimentale, on peut citer à titre d'exemple de spectromètre de masse utilisable dans le cadre de l'invention, le système ClinProt^TM développé par la société Bruker Daltonics, qui utilise des microparticules magnétiques avec une surface fonctionnalisée.

Dans le cadre des dosages par comparaison de profils, plusieurs méthodes de détection du signal peuvent être utilisées, seules ou en couplage avec des procédés séparatifs (Technologies TOF, laser-MS et MS-MS, ESI-MS, LC-MS, MALDI, tandem, Q-Trap etc.... SELDI-MS, SELDI-MS-MS, chémoluminescence (Mesoscale, Roche diagnostics), technologie Elecsys, fluorescence, ICAT, signal piezoélectrique, signal optique comme la "surface plasmon resonance, S.P.R."), pour mettre en évidence et doser les molécules issues des profils, soit sous forme native, soit sous forme ionique (1+, 2+, 3+, 4+, 5+ etc...), applicables aux protéines, aux glycoprotéines et aux lipoprotéines.

• Dosage par immuno-réaction (*Immuno Assay,* ou IA) : différentes technologies, connues de l'homme du métier, peuvent être envisagées, telles que le *Sandwich ImmunoAssay* (Sandwich IA), l'interaction de surface (*Surface Plasma Resonance,* ou SPR), ou l'immuno-précipitation (par exemple par la technologie *Q-MAP* développée par Atto-Lab).

L'approche *Sandwich IA* se base sur la double reconnaissance du marqueur - ou antigène par des anticorps spécifiques : les anticorps dits "de capture" immobilisés sur le support choisi (plaque de micro-titration, bille magnétique...), et les anticorps dits "de détection", en solution et couplés à une molécule réactive, responsable de l'émission du signal de liaison (directement ou indirectement). Ce jeu d'anticorps spécifiques non recouvrant (Pair Matched *Antibodies)* constitue les réactifs communs aux différents systèmes de détection actuels.

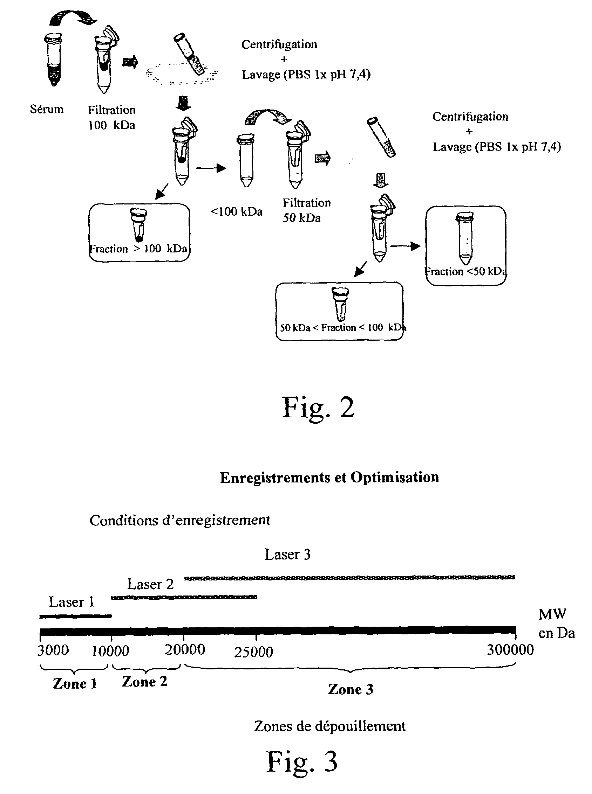

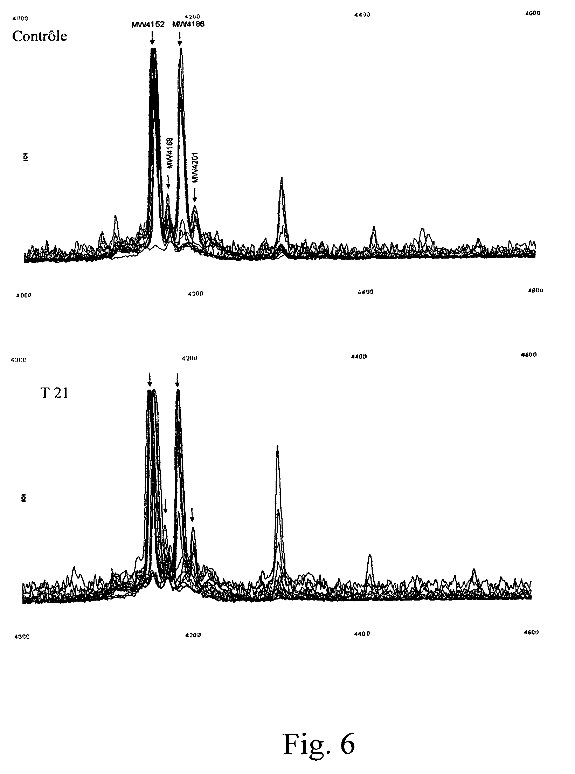

Alternativement, la reconnaissance de l'antigène peut se faire en utilisant des aptamères, qui sont des séquences oligonucléotidiques (ADN ou ARN) simples brins sélectionnées *in vitro* pour leur capacité de liaison à une molécule donnée ; cette sélection peut se faire, par exemple, par la méthode SELEX (*Systematic Evolution of Ligands by Exponential Enrichment*) telle que décrite dans le Brevet US 5,270,163. Un aptamère fonctionnalisé peut, par exemple, être utilisé à la place d'un anticorps secondaire dans un test immunologique de type "Sandwich *ELISA",* comme décrit par Sumedha Jayasena (*Clinical Chemistry* 1999, 45(9) : 1628-50, Fig. 6). Les aptamères peuvent faire l'objet d'une optimisation par différentes modifications (Sumedha Jayasena, supra, Fig. 2). Il est notamment possible de modifier les aptamères de telle sorte que l'activation par UV après liaison de leur cible assure la liaison covalente de cette cible au support (*photoaptamères*).

Les différentes composantes d'un dispositif diagnostic de type immuno-réaction dépendent également du système de détection utilisé. Certains réactifs et supports utilisables pour mettre en oeuvre l'invention sont résumés dans le Tableau 1 ci-dessous, ainsi que des exemples d'instrumentation adaptés.

| Tableau 1 : les différentes composantes d'un dispositif diagnostic de type IA | | | |
|---|---|---|---|
| Système de détection | Réactifs (sauf anticorps ou aptamères de capture et de détection et tampons associés) | Support | Instrumentation (Photomultiplicateurs et caméras CCD pour les IFA, CLIA et ECLIA) |
| RIA *(Radioimmuno Assay)* | Radiolsotopes ($^{125}$I) | Microplaques, Microbilles, Microbilles magnétiques. | Technicon Star System... |
| IFA *(Immunofluorescent Assay), dont les techniques FP (Fluorescence Polarisation), TRF (Time Resolved Fluorescence) et FRET (Fluorescence Resonance Energy Transfer)* | fluorophores (Aqualite, Lanthanes, FITC, PE...) | | DELFIA (Perkin)... |
| CLIA *(Chemiluminescence Assay)* | Enzymes (AP, HRP... ) Substrat (Acridium, Cryptate...) | | Advia Centaur (Brahms), AxSym (Abbott), Immulite 2000 (DPC) |
| ECLIA *(Electrochemiluminescence Assay)* | Substrat (Ruthenium) | | Elecsys 2010 (Roche)... |

Des puces à protéines peuvent être conçues pour déterminer des profils d'expression protéique choisis, impliquant les marqueurs souhaités. Cette technologie est présentée dans l'article de revue de Sydor et Nock (Proteome Science, 2003, June 10; 1(1) : 3). Bien entendu, l'homme du métier peut améliorer les technologies présentées dans cet article en utilisant ses connaissances générales dans ce domaine.

A titre d'exemples particuliers de technologies d'immuno-réaction innovantes utilisables dans le cadre de l'invention, on citera la technologie MAP (Luminex Corp), les *Bio Bar Codes* (Nanotechnologies Inc.), les marqueurs LumiPhos 530 et 480 (Lumigen), et la *RCA (Rolling-circle* Amplification).

La technologie MAP (Luminex Corp) allie cytométrie de flux et microparticules sous un format de type IFA (*Immunofuorescent Assay)*. Cette technologie propose une analyse en double fluorescence. Le premier système de détection permet de capter la fluorescence émise par les billes de capture des marqueurs (à une bille correspondent un marqueur et une fluorescence) ; le second permet quant à lui la mesure de la fluorescence associée aux anticorps de détection, c'est-à-dire à la quantification des marqueurs.

Les Bio Bar *Codes* (Nanotechnologies Inc.) allient nanoparticules et scanométrie (Argent), introduisant un nouveau système de détection basé sur l'hybridation de sondes d'acides nucléiques uniques (identification des marqueurs) (*Nanoparticle-Based Bio-Bar Codes for the Ultrasensitive Detection of Proteins,* Jwa Min Nam and al., *Science* 2003).

Les marqueurs LumiPhos 530 et 480 (Lumigen) combinent les systèmes de détections chemiluminescent et fluorescent, assurant par là une optimisation du signal.

La RCA (Rolling-circle Amplification) permet la détection de protéines avec une sensibilité de l'ordre de la zeptomole ($10^{-21}$ moles). Cette technologie, décrite par Schweitzer *et al* (Proc Natl Acad Sci U S A. 2000 Aug 29;97(18):10113-9), implique la polymérisation d'une longue chaîne d'ADN simple brin fixée à l'analyte (par exemple, un anticorps), à partir d'une matrice d'ADN circulaire. Cette technologie permet de minimiser les volumes d'échantillons et l'utilisation de matériels coûteux (anticorps monoclonaux et enzymes).

On notera également la possibilité d'utilisation de systèmes d'amplification du signal comme autre catégorie de réactif dans le cadre notamment des IFA et CLIA (ex : biotine/avidine).

La présente invention porte également sur une trousse de diagnostic prénatal d'anomalies chromosomiques, comprenant des moyens appropriés pour mettre en oeuvre un des procédés décrits plus haut.

L'homme du métier est capable de sélectionner, suivant les techniques de dosage et de détection qu'il souhaite utiliser pour mettre en oeuvre l'invention, les éléments à intégrer dans une telle trousse de diagnostic : anticorps spécifiques, anticorps secondaires, aptamères, réactifs de détection (composés fluorescents, enzymes, anticorps modifiés pour utiliser la technologire RCA, ...) , etc... Il peut choisir au moins certains de ces éléments parmi les éléments mentionnés ci-dessus, et compléter la trousse, le cas échéant, à l'aide de ses connaissances générales et d'ouvrages de référence sur la purification, le dosage, ou la détection de molécules biologiques.

Une trousse de diagnostic prénatal selon l'invention peut par exemple comporter des anticorps spécifiques et/ou des aptamères d'un ou plusieurs marqueurs cités ci-dessus. Bien entendu, dans une trousse qui contient plusieurs anticorps ou aptamères spécifiques de marqueurs, chaque anticorps ou aptamère est spécifique d'un seul marqueur. Lorsque ces composés sont en solution, chaque solution est de préférence spécifique d'un seul marqueur, ou d'un groupe de marqueurs associés (par exemple, les défensines HND-1, HND-2 et HND-3). A titre d'exemples d'anticorps qui peuvent être inclus dans une trousse selon l'invention, on peut citer les anticorps anti-HND tels que ceux commercialisés par Tebu-Bio SA (39 rue de Houdan, F-78612 Le Perray en Yvelines) sous la référence 038NCL-DEFENSIN *(Anti Neutrophil Defensins* Mouse *IgG1 Clone D21,* 1 ml).

Avantageusement, une trousse de diagnostic selon l'invention comprend, pour au moins un des marqueurs de l'invention, un jeu d'anticorps spécifiques non recouvrant, c'est-à-dire au moins deux anticorps capables de reconnaître simultanément le marqueur en question, ce qui permet de réaliser des tests d'immuno-réaction de type "sandwich".

Les anticorps, en particulier les anticorps dits "de capture", peuvent être immobilisés sur un support, et les anticorps dits "de détection" couplés à une molécule permettant la détection. Des exemples non limitatifs de supports et de molécules permettant la détection, utilisables dans le cadre de l'invention, sont mentionnés ci-dessus.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- la figure 1 illustre la stratégie expérimentale utilisée ;
- la figure 2 illustre une préparation schématique des échantillons ;
- la figure 3 résume les différentes conditions d'enregistrement, en fonction de la masse moléculaire des protéines à détecter ;
- la figure 4 illustre le schéma expérimental utilisé dans les études E1, E2, E3, E4 et E6 ;
- la figure 5 représente les spectres obtenus sur la zone spectrale 3000-20000 Daltons, en vues globales, pour le groupe contrôle, le groupe T21 et le groupe T18 ;
- les figures 6-8 illustrent les spectres centrés sur la zone 4000 Daltons (figure 6 : spectres en vues globales ; figure 7 : pseudo-gels ; figure 8 : spectres en vues décalées) ;
- les figures 9-11 illustrent les spectres centrés sur la zone 5000-6000 Daltons (figure 9 : spectres en vues globales ; figure 10 : pseudo-gels ; figure 11 : vues décalées) ;
- les figures 12 à 14 illustrent les spectres centrés sur la zone spectrale 3000-10000 Daltons (figure 12 : spectres en vues globales ; figure 13 : pseudo-gels ; figure 14 : vues décalées) ;
- la figure 15 illustre les spectres centrés sur la zone 3000-4000 Daltons (spectres en vues globales) ;
- les figures 16 à 18 illustrent les spectres centrés respectivement sur les zones 4000-5000 Daltons, 5000-7000 Daltons et 7000-10000 Daltons (spectres en vues globales) ;
- la figure 19 illustre les spectres en vues globales, normalisés et superposés par catégorie clinique, pour le groupe contrôle, le groupe T21, et le groupe T18, sur la zone spectrale 3000-10000 Daltons (étude E6) ;
- les figures 20 et 21 illustrent, pour la même zone spectrale que celle de la figure 19, les vues en pseudo-gels (figure 20) et les vues décalées (figure 21) uniquement pour le groupe contrôle et le groupe T21 ;

- les figures 22-24 présentent les spectres sur la zone spectrale 3000-2000 Daltons, respectivement en vues globales (spectres normalisés et superposés par catégorie clinique) pour les groupes contrôle, T21 et T18, en vues pseudo-gels pour les groupes contrôle et T21, et en vues décalées pour les mêmes groupes (étude E6) ;
- la figure 25 montre les spectres centrés sur la zone 6500, après normalisation par rapport à l'intensité du cluster PM6622 (études E4 et E5). Cette figure montre la superposition des spectres pour les catégories contrôle et T21 (étude E6) ;
- la figure 26 montre les spectres centrés sur la zone 6500 après normalisation sur le pic PM6622, (spectres normalisés et superposés par catégorie clinique), pour les groupes T21, contrôle, et T18. Sur cette figure, le pic référencé N correspond au cluster PM6622 et sert à normaliser les spectres ;
- les figures 27 à 30 montrent des exemples de statistiques de distribution des intensités de différents clusters, pour les groupes contrôle et T21 :

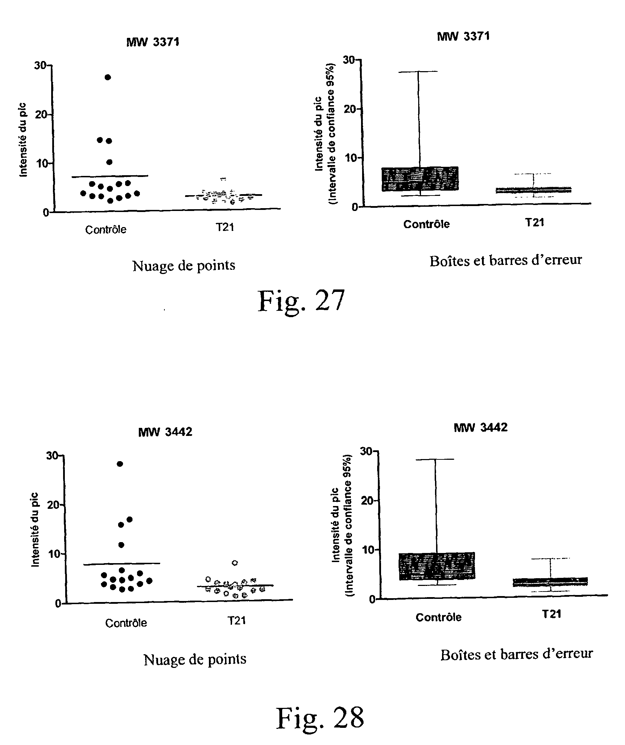

  • figure 27 : Cluster PM3371, phase séparative anionique, tout pH > 4.
  • figure 28 : Cluster PM3442, phase séparative anionique, tout pH > 4.

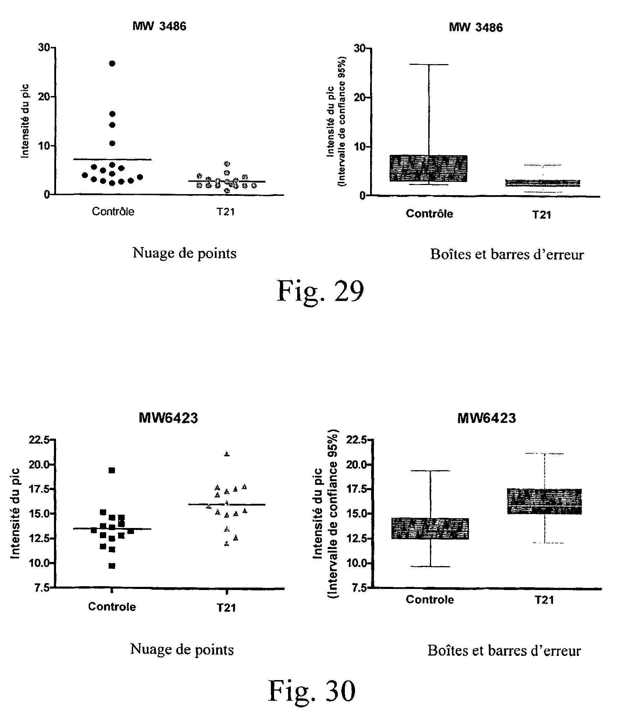

  • figure 29 : Cluster PM3486, phase séparative anionique, tout pH > 4.
  • figure 30 : Cluster PM6423, phase séparative hydrophobique, normalisation sur l'intensité du cluster PM6620 ;

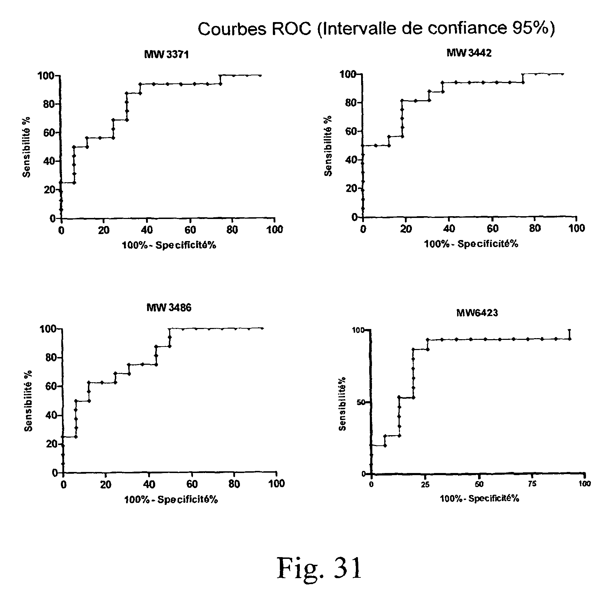

- la figure 31 montre les courbes ROC pour les marqueurs de poids moléculaires 3371, 3442, 3486, et 6423 Da ;

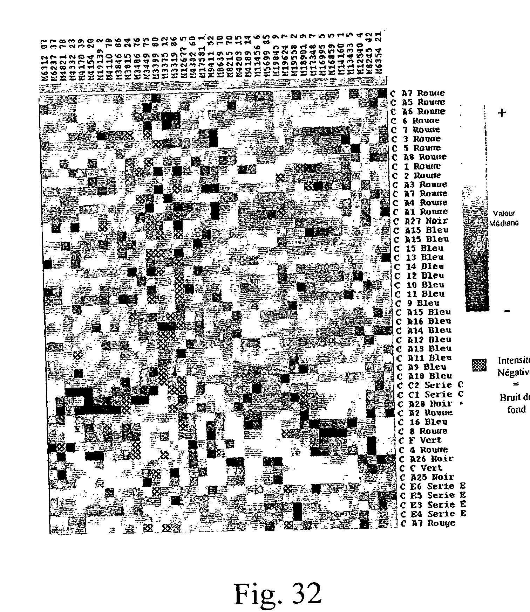

- la figure 32 montre le résultat d'une construction avec les logiciels Genecluster Version 1999 (Eisen M.) et Treeview Version 1.60 (Eisen M.) (Classification hiérarchique sur Jeu de Données de l'étude E6 (IC 95%), Surface Anionique Q10) ;

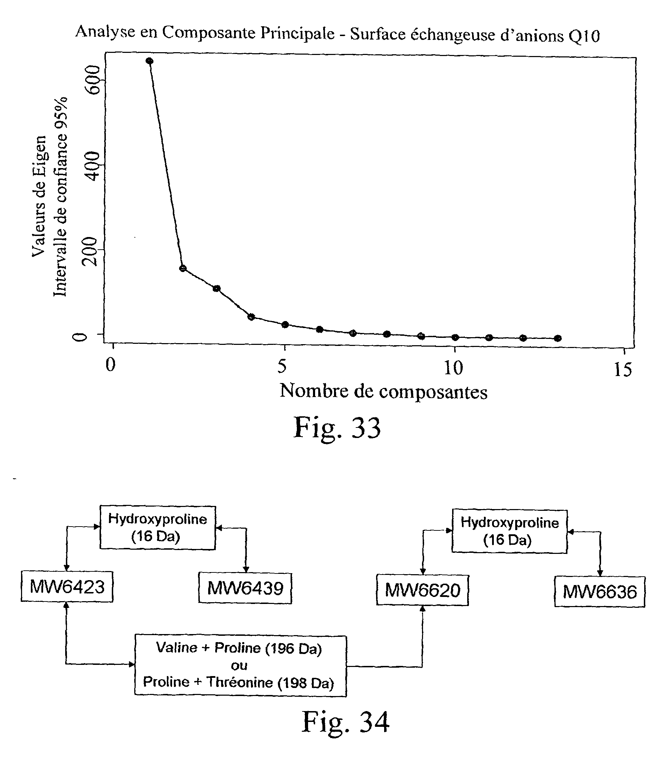

- la figure 33 illustre l'analyse en composante principale pour les défensines (surface échangeuse d'anions Q10) ;
- la figure 34 illustre la procédure d'identification des clusters PM6423, PM6439, PM6620 et PM6636 (PM est représenté par MW dans cette figure).

Il doit être bien entendu, toutefois, que ces exemples sont donnés uniquement à titre d'illustration de l'objet de l'invention, dont ils ne constituent en aucune manière une limitation.

**EXEMPLES**

**A - MATERIELS ET METHODES**

**A - 1 Echantillons Biologiques - Population d'Etude**

Recueillis rétrospectivement et prospectivement dans un cadre multicentrique, les échantillons biologiques de sang maternel ont été prélevés selon les recommandations et pratiques actuelles entre la 14$^{ème}$ et la 25$^{ème}$ semaine de grossesse, acheminés en procédure centralisée, conservés à + 4°C selon les procédures actuelles en vigueur, aliquotés et congelés à - 20°C (échantillons historiques).
Dans le but de vérifier la faisabilité en routine, la robustesse de la méthode, ainsi que la comparabilité des résultats entre différentes conditions de congélation, et en regard des futures procédures et recommandations du domaine, de nouveaux échantillons ont été recueillis sur la même base méthodologique clinique, acheminés à + 4°C, mais aliquotés et congelés directement à - 80°C (échantillons contemporains).
Dans tous les cas, les échantillons ont été stockés et congelés à -80°C.
Les annotations suivantes, constituant les données cliniques et biologiques de chaque échantillon, ont été recueillies :

- Numéro de dossier,
- Date du prélèvement,
- Âge et poids de la mère
- Semaine de grossesse ajustée au nombre de jours de la semaine au prélèvement = nombre de jours de gestation
- Sexe du foetus,
- Marqueurs Biologiques : hCG en UI/ml, uE3 en pg/ml, $\alpha$-foetoprotéine en UI/ml, les 3 convertis en multiple de la médiane (MoM = valeur mesurée/valeur médiane),
- Risque, calculé en fonction de l'âge de la femme enceinte et de ses données en MoM,
- Indication (âge, marqueurs, signes cliniques échographiques)
- Résultat du caryotype foetal (techniques conventionnelles)
- Issue de la grossesse (naissance, IMG, MFIU mort foetale in utéro, éventuellement résultats anatomopathologiques).

Les échantillons dont l'issue est connue puisque les études ont été menées rétrospectivement ont été répartis en plusieurs groupes, dont un groupe de mères porteuses d'enfants normaux et un groupe de mères porteuses d'enfants atteints du Syndrome de Down (T21). La robustesse des études est par ailleurs renforcée par la présence de mères porteuses d'autres anomalies (Trisomie 18 ou Syndrome d'Edwards, Syndrome de Turner, Délétion terminale du bras long du chromosome 7 (del 7q) ) .

Dans le but de contrôler la validité interne des études, les échantillons ont été analysés en aveugle ou double aveugle dans les études cas-contrôle (afin de s'affranchir des biais de suivi pendant les analyses et des biais d'attrition).

Toutes les études rétrospectives l'ont été sur échantillons appariés *(cases and controls matched),* afin de s'affranchir des biais de confusion et rendre comparables les études. Un suivi longitudinal des expériences a été réalisé afin de contrôler la variabilité expérimentale (instrumentale et humaine). La taille des populations étudiées et la puissance requise assurent la validité externe des résultats.

**A - 2 Matériels et Méthodes d'Analyse des Protéines**

Toutes les recherches ont été réalisées en environnement de bonnes Pratiques de Laboratoire, ou B.P.L.(G.L.P.), en particulier:

Matériels et Consommables

Un spectromètre de masse de type PBS II c équipé du logiciel d'acquisition des spectres ProteinChip® Software V.3.1.1 et du Bio Marker Wizard (B.M.W) Software Version 3.1. (Ciphergen Biosystems Inc., Fremont, CA, USA) a été utilisé.

Les inventeurs ont utilisé différentes surfaces de purification en approche avec et sans fractionnement, avec ou sans déplétion en albumine, en mode "reverse phase", en mode ciblé avec des anticorps, en mode étude des phosphorylations comme des glycosylations, dont les chimies de surface, d'interaction, et les matrices utilisées pour l'ionisation des protéines sont récapitulées dans le Tableau 2 ci-dessous. Les références indiquées sont, pour chaque surface, les références du fournisseur (Ciphergen Biosystems Inc., Fremont, CA, USA).

| Tableau 2 | | | |
|---|---|---|---|
| **Références** | **Propriétés des Surfaces** | **Chimie de surface** | **Catalogue** |
| C553-0028 | Hydrophobe & Cationique - H4 | Groupements C16 + Groupes carboxylate | Janvier 2003 |
| C553-0065 C554-0065 | Hydrophobe - H50 | Chaines C6 à C12 | Septembre 2003 |
| C553-0043 | Phase normale - NP20 | Oxide de silice | Janvier 2003 |
| C553-0045 | PS20 | Groupements Epoxy | Janvier 2003 |
| C553-0027 C554-0052 | Anionique - SAX2 / LSAX30 | Groupe Amine quaternaire | Janvier 2003 |
| C573-0080 | Anionique - Q10 | Groupe Amine quaternaire | Septembre 2003 |
| C553-0026 C554-0056 | Cationique - WCX2 / Groupe | Groupe carboxylate carboxylate | Janvier 2003 |
| C553-0075 | Cationique - CM10 | Groupe carboxylate | Septembre 2003 |
| C553-0022 C554-0057 | Métallique - IMAC3 / IMAC40 | Acide nitrilotriacetique | Janvier2003 |
| C300-0001 | Matrice - CHCA | Acide alpha-cyano-4-hydroxy cinnamic | Janvier 2003 |
| C300-0003 | Matrice - EAM | | Janvier 2003 |
| C300-0002 | Matrice - SPA | Acide sinapinique | Janvier 2003 |

La résolution et la précision en masse ont été vérifiées mensuellement, avant, pendant et après chaque expérience, dans le cadre de procédures de contrôle qualité mettant en oeuvre des tests sur puce dorée en mode MALDI et sur puces à oxyde de silice en mode SELDI et utilisant les calibrants du Tableau 3 ci-après. Les références indiquées sont les références du fournisseur (Ciphergen Biosystems Inc., Fremont, CA, USA).

| Tableau 3 | |
|---|---|
| Références | Calibrants |
| C100-0004 | Cytochrome C équin, Myoglobine de muscle équin, GAPDH de lapin, Albumine sérique bovine, Bêta-Galactosidase de *E. coli* |
| C100-0007 | Hirudine BHVK, Cytochrome C bovin, Myoglobine de muscle équin, Anhydrase carbonique de globules rouges bovins, Enolase de *S. cerevisiae,* Albumine de sérum bovine IgG bovins |
| C100-0005 | Arg8-Vasopressine, Somatatostatine Chaîne bêta d'insuline bovine, Insuline humaine, Hirudine BHVK |
| C100-0002 | Insuline bovine |
| C100-0001 | Béta-Lactoglobuline A bovine, Peroxydase de raifort |

Les conditions expérimentales utilisées en routine dans un mode particulier (tampons, matrices, chimies de surfaces, pH de travail, Intensités laser) sont toujours réalisées en environnement contrôlé (20°C-25°C) ; elles sont listées dans le Tableau 4 ci-après.

| Tableau 4 | | | | | | | |
|---|---|---|---|---|---|---|---|
| Référence | Chimie de Surface | Matrice | Tampon Hydratation (50 à 100µl) | pH | Tampon Incubation (100µl) | Tampon Lavage (100µl) | Laser (Cf. Fig. 3) |
| C553-0028 | C 16 + Carboxylate | SPA | acetonitrile 5% | NA | acetonitrile 5% | acetonitrile 5% | Int 1 |
| C553-0028 | C 16 + Carboxylate | SPA | acetonitrile 5% | NA | acetonitrile 5% | acetonitrile 5% | Int 2 |
| C553-0028 | C 16 + Carboxylate | SPA | acetonitrile 5% | NA | acetonitrile 5% | acetonitrile 5% | Int 3 |
| C553-0065 / C554-0065 | C6 à C12 | CHCA | acetonitrile 50% | 7,4 physio | PBS 1X + 0,1% TFA | PBS 1X + 0,1% TFA | Int 1 |
| C553-0065 / C554-0066 | C6 à C13 | CHCA | acetonitrile 50% | 7,4 physio | PBS 1X + 0,1 % TFA | PBS 1X + 0,1 % TFA | Int 2 |
| C553-0065 / C554-0067 | C6 à C14 | CHCA | acetonitrile 50% | 7,4 physio | PBS 1X + 0,1% TFA | PBS 1X + 0,1 % TFA | Int 3 |
| C553-0022 | Acide Nitriloacétique + Cu | CHCA | CuSO4 100nM | 7,4 physio | PBS 1X + 0,1 Triton X100 | PBS 1X | Int 1 |
| C553-0023 | Acide Nitriloacétique + Cu | CHCA | CuSO4 100nM | 7,4 physio | PBS 1X + 0,1 Triton X100 | PBS 1X | Int 2 |
| C554-0057 | Acide Nitriloacétique + Hydrophobicité + Cu | CHCA | CuSO4 100nM | 7,4 physio | PBS 1X + 0,1 Triton X100 | PBS 1X | Int 1 |
| C554-0057 | Acide Nitriloacétique + Hydrophobicité + Cu | CHCA | CuSO4 100nM | 7,4 physio | PBS 1X + 0,1 Triton X100 | PBS 1X | Int 2 |
| C554-0057 | Acide Nitriloacétique + Hydrophobicité + Cu | CHCA | CuSO4 100nM | 7,4 physio | PBS 1X + 0,1 Triton X100 | PBS 1X | Int 3 |
| C554-0057 | Acide Nitriloacétique + Hydrophobicité + Cu | CHCA | CuSO4 100nM | 7,4 physio | PBS 1X + 0,1 Triton X100 | PBS 1X | Int 4 |
| C554-0057 | Acide Nitriloacétique + Hydrophobicité + Ni | CHCA | NiSO4 100nM | 7,4 physio | PBS 1X + 0,1 Triton X100 | PBS 1X | Int 1 |
| C554-0057 | Acide Nitriloacétique + Hydrophobicité + Ni | CHCA | NiSO4 100nM | 7,4 physio | PBS 1X + 0,1 Triton X100 | PBS 1X | Int 2 |
| C554-0057 | Acide Nitriloacétique + Hydrophobicité + Ni | CHCA | NiSO4 100nM | 7,4 physio | PBS 1X + 0,1 Triton X100 | PBS 1X | Int 3 |
| C554-0057 | Acide Nitriloacétique + Hydrophobicité + Ni | CHCA | NiSO4 100nM | 7,4 physio | PBS 1X + 0,1 Triton X100 | PBS 1X | Int 4 |
| C554-0052 | Groupe Amine Quaternaire + Latex | SPA | PBS 1X | 7,4 physio | PBS 1X | PBS 1X | Int 1 |
| C554-0052 | Groupe Amine Quaternaire + Latex | SPA | PBS 1X | 7,4 physio | PBS 1X | PBS 1X | Int 2 |
| C554-0052 | Groupe Amine Quaternaire + Latex | SPA | PBS 1X | 7,4 physio | PBS 1X | PBS 1X | Int 3 |
| C554-0052 | Groupe Amine Quaternaire + Latex | SPA | Phosphate Citrate 20nM | 4 | Phosphate Citrate 20nM+0,1% Triton X100 | Phosphate Citrate 20nM | Int 1 |
| C554-0052 | Groupe Amine Quaternaire + Latex | SPA | Phosphate Citrate 20nM | 4 | Phosphate Citrate 20nM+0,1% Triton X100 | Phosphate Citrate 20nM | Int 2 |
| C554-0052 | Groupe Amine Quaternaire + Latex | SPA | Phosphate Citrate 20nM | 4 | Phosphate Citrate 20nM+0,1% Triton X100 | Phosphate Citrate 20nM | Int 3 |
| C554-0052 | Groupe Amine Quaternaire + Latex | SPA | Tris Citrate 20nM | 6 | Tris Citrate + 0,1% Triton X100 | Tris Citrate 20nM | Int 1 |
| C554-0052 | Groupe Amine Quaternaire + Latex | SPA | Tris Citrate 20nM | 6 | Tris Citrate + 0,1% Triton X100 | Tris Citrate 20nM | Int 2 |
| C554-0052 | Groupe Amine Quaternaire + Latex | SPA | Tris Citrate 20nM | 6 | Tris Citrate + 0,1% Triton X100 | Tris Citrate 20nM | Int 3 |
| C554-0056 | Groupe Carboxylate + Latex | SPA | PBS 1X | 7,4 physio | PBS 1X | PBS 1X | Int 1 |
| C554-0056 | Groupe Carboxylate + Latex | SPA | PBS 1X | 7,4 physio | PBS 1X | PBS 1X | Int 2 |
| C554-0056 | Groupe Carboxylate + Latex | SPA | PBS 1X | 7,4 physio | PBS 1X | PBS 1X | Int 3 |
| C554-0056 | Groupe Carboxylate + Latex | CHCA | Phosphate Citrate 20nM | 4 | Phosphate Citrate 20nM+ 0,1% Triton X100 | Phosphate Citrate 20nM | Int 1 |
| C554-0056 | Groupe Carboxylate + Latex | CHCA | Phosphate Citrate 20nM | 4 | Phosphate Citrate 20nM+ 0,1% Triton X100 | Phosphate Citrate 20nM | Int 2 |
| C554-0056 | Groupe Carboxylate + Latex | CHCA | Phosphate Citrate 20nM | 4 | Phosphate Citrate 20nM+ 0,1% Triton X100 | Phosphate Citrate 20nM | Int 3 |
| C554-0056 | Groupe Carboxylate + Latex | SPA | Phosphate Citrate 20nM | 4 | Phosphate Citrate 20nM+ 0,1% Triton X100 | Phosphate Citrate 20nM | Int 1 |
| C554-0056 | Groupe Carboxylate + Latex | SPA | Phosphate Citrate 20nM | 4 | Phosphate Citrate 20nM+ 0,1% Triton X100 | Phosphate Citrate 20nM | Int 2 |
| C554-0056 | Groupe Carboxylate + Latex | SPA | Tris Citrate 20nM | 6 | Tris Citrate + 0,1% Triton X100 | Tris Citrate 20nM | Int 1 |
| C554-0056 | Groupe Carboxylate + Latex | SPA | Tris Citrate 20nM | 6 | Tris Citrate + 0,1% Triton X100 | Tris Citrate 20nM | Int 2 |
| C554-0056 | Groupe Carboxylate + Latex | SPA | Tris Citrate 20nM | 6 | Tris Citrate + 0,1% Triton X100 | Tris Citrate 20nM | Int 3 |
| C553-0043 | Oxyde de Silice | SPA | Hépès 1mM | 7,4 | Hépès 1mM | Hépès 1mM | Int 1 |
| C553-0043 | Oxyde de Silice | SPA | Hépès 1mM | 7,4 | Hépès 1mM | Hépès 1mM | Int 2 |
| C553-0043 | Oxyde de Silice | SPA | Hépès 1mM | 7,4 | Hépès 1mM | Hépès 1mM | Int 3 |
| C553-0026 | Groupe Carboxylate | CHCA | PBS 1X | 7,4 physio | PBS 1X | | Int 1 |
| C553-0026 | Groupe Carboxylate | CHCA | PBS 1X | 7,4 physio | PBS 1X | | Int 2 |
| C553-0026 | Groupe Carboxylate | CHCA | PBS 1X | 7,4 physio | PBS 1X | | Int 3 |
| C553-0026 | Groupe Carboxylate | CHCA | Tris Citrate 20nM | 5 | Tris Citrate 20nM | Tris Citrate 20nM | Int 1 |
| C553-0026 | Groupe Carboxylate | CHCA | Tris Citrate 20nM | 5 | Tris Citrate 20nM | Tris Citrate 20nM | Int 2 |
| C553-0026 | Groupe Carboxylate | CHCA | Tris Citrate 20nM | 5 | Tris Citrate 20nM | Tris Citrate 20nM | Int 3 |
| C553-0026 | Groupe Carboxylate | CHCA | Tris-HCl 50nM | 9 | Tris-HCl 50nM | Tris-HCl 50nM | Int 1 |
| C553-0026 | Groupe Carboxylate | CHCA | Tris-HCl 50nM | 9 | Tris-HCl 50nM | Tris-HCl 50nM | Int 2 |

Pour les études ciblées, les anticorps monoclonaux suivants ont été utilisés :

- Anti-hCG et Bêta hCG, Référence RDI-CBL75 (*RDI Research Diagnostics Inc.*)
- Anti-Alpha-Foetoprotéine humaine, Référence RDI-TRK4F16-4A3 *(RDI Research Diagnostics Inc.),*
- Anti-PAPP-A, Référence RDI-TRK4P41-10E2 (RDI Research *Diagnostics Inc.)*

Stratégie Expérimentale utilisée

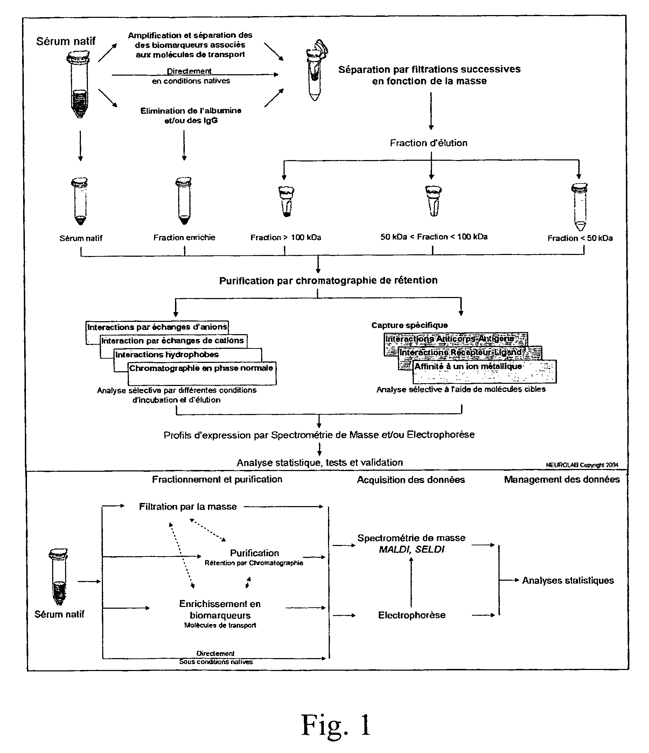

La stratégie expérimentale utilisée est schématisée à la figure 1.

Préparation des Echantillons

*Sérums bruts ou natifs*

Les échantillons conservés congelés depuis leur collecte, sont décongelés sur lit de glace pour analyse directe et/ou après purification préalable.

*Déplétion*

Les échantillons sont partiellement déplétés en protéines abondantes telles que l'albumine ou les immunoglobulines.

*Enrichissement des protéines associées aux molécules de transport*

Les protéines de transport et les molécules qui leur sont associées sont purifiées, augmentant de fait la concentration relative des molécules transportées dans la fraction de l'échantillon analysée.

*Sérums fractionnés* en *fonction de la masse native*

Les échantillons bruts ou partiellement déplétés ou enrichis sont fractionnés en condition native par séparation en fonction de la masse des protéines.

Synthèse de *la préparation* des échantillons

La préparation des échantillons est présentée sous forme schématique à la figure 2.

Procédés de purification

Les échantillons ont été préparés selon différents degrés de fractionnement et purifiés par chromatographie de rétention sur phase solide de surface et/ou chromatographie liquide.

*Purification* de type biochimique

Les propriétés biochimiques propres à chaque protéine sont utilisées afin de les séparer les unes des autres selon leurs charges apparentes de surface (propriétés ioniques) et leur hydrophobicité. Parallèlement, leur concentration relative au sein de l'échantillon et/ou de la fraction analysée de l'échantillon est étudiée. Quatre approches différentes sont utilisées :

*Chromatographie* en phase *normale*

Cette approche chromatographique reposant sur l'utilisation d'oxyde de silice, permet de vérifier la qualité d'un échantillon et d'apprécier la concentration relative d'une protéine. L'association se fait par l'intermédiaire d'arginines, de lysines, de sérines et de thréonines.

*Chromatographie échangeuse d'anions*

Un groupement fonctionnel chargé positivement (par exemple : amines quaternaires) est utilisé pour séparer les molécules ayant un caractère anionique avéré (chargées négativement). Deux paramètres variables sont généralement explorés : le pH auquel la phase d'association-incubation a lieu et le degré de stringence de l'éluant utilisé.

*Chromatographie échangeuse de cations*

Un groupement fonctionnel chargé négativement (par exemple : groupement carboxylate) est utilisé pour séparer les molécules ayant un caractère cationique avéré (chargées positivement). Comme pour la chromatographie échangeuse d'anions, différentes combinaisons expérimentales sont appliquées aux phases d'association et d'élution.

*Interactions hydrophobiques*

Les interactions hydrophobes libèrent l'eau structurée entourant des secteurs hydrophobes des biomolécules. Ceci a pour conséquence d'accroitre l'entropie, rendant les interactions thermodynamiquement favorables. Une chromatographie utilisant une chaîne de carbones (par exemple : $C_{18}$) peut être utilisée. Les conditions d'association et d'élution dépendent du degré d'hydrophobicité de l'éluant utilisé.

*Purification ciblée*

Deux schémas expérimentaux ont été utilisés pour une approche ciblée de la purification de protéines. Dans un premier cas, les caractéristiques biologiques des protéines à purifier révèlent des capacités d'association à un ligand métallique et/ou chimique. Dans le second cas, l'identité de la protéine à isoler étant connue, permet d'employer une stratégie immunitaire, faisant dès lors appel à un anticorps spécifique de la protéine ciblée.

➢ Capture par affinité avec un ligand

Il s'agit d'une stratégie de purification reposant sur la forte affinité d'une protéine pour un ligand chimique connu. Seules les protéines affines de ce ligand seront retenues sur la surface spécifiquement préparée à cet usage.

➢ Interactions anticorps-antigène

Il s'agit d'une stratégie de purification reposant sur la connaissance de la protéine ciblée et l'existence d'anticorps spécifiques de la cible. La très forte affinité existant entre l'anticorps et la protéine contre laquelle il est dirigé est utilisée pour séparer la cible des autres protéines de l'échantillon. La purification simultanée de facteurs associés à la cible est possible.

Modes et Procédés d'Acquisition des Données

*Introduction*

Différents modes d'analyse des données sont utilisés. Les résultats issus des phases de purification par chromatographie liquide peuvent être visualisés directement lors des différentes phases d'élution des protéines par spectroscopie d'absorption, ou indirectement, par séparation par mobilité électrophorétique en conditions dénaturantes ou encore par spectrométrie de masse.

*Spectroscopie d'absorption*

L'enregistrement des données a lieu en sortie de colonne de chromatographie par insertion d'un spectrophotomètre UV ($\lambda$ = 280 nm) en série avant le collecteur. L'absorption à cette longueur d'onde est principalement due au tryptophane et à la tyrosine.

*Electrophorèse dénaturante*

Les différentes fractions de protéines issues des étapes de fractionnement et de purification sont dénaturées par action d'agents dénaturants (détergents, etc....) et dégradation thermique. Les protéines dénaturées migrent dans une phase poreuse (le gel) sous contrainte électrostatique. Leurs caractéristiques physico-chimiques (pI, masse, etc...) assurent une bonne séparation des différents produits.

*Spectrométrie de masse*

Les protéines ionisées sont séparées en fonction de leur masse par les technologies suivantes :

➢ Spectrométrie de masse par désorption laser

Les protéines sont déposées sur une surface active (spectrométrie de masse SELDI - Surface *Enhanced Laser Desorption Ionisation),* ou passive (spectrométrie de masse MALDI - *Matrix Assisted Laser Desorption* Ionisation), associées à une matrice chimique permettant, sous vide, leur ionisation par un faisceau laser. Une fois ionisées, elles sont accélérées et dirigées sous contrainte électrostatique vers un détecteur placé à l'extrémité d'un tube sous vide. La vitesse à laquelle elles atteignent le détecteur (TOF - *Time of* Flight) est proportionnelle à la racine carrée de leur masse.

➢ Spectrométrie de masse par électrospray

Les protéines sont nébulisées puis ionisées sous l'action d'un gaz inerte en passant dans une aiguille très fine fortement chargée. L'analyseur est le plus souvent un "TOF" du même type que pour la spectrométrie de masse par désorption laser.

*Méthodologie d'Enregistrement* et *d'Analyse des Spectres : Conditions Expérimentales d'enregistrement, d'optimisation et d'annotation*

Les enregistrements et l'analyse des spectres sont effectués selon différents protocoles, qui dépendent de la surface et de l'appareillage (intensité laser, sensibilité en masse du détecteur, gain du détecteur, réglage du déflecteur). Ces paramètres sont optimisés en fonction de la zone de la gamme de masse à analyser.
Les facteurs de normalisation utilisés sont principalement le *Total Ion Courant* (TIC), certains marqueurs ayant une normalisation spécifique.
La figure 3 résume les différentes conditions d'enregistrement, en fonction de la masse moléculaire des protéines à détecter.
Lors des étapes préliminaires à l'analyse, les spectres et les clusters associés sont annotés selon deux procédures complémentaires, l'une automatique, l'autre manuelle relevant de l'art du traitement du signal. Les deux procédures sont alors confrontées entre elles.

## B - GENERALITES SUR LE MANAGEMENT DES DONNEES ET L'ANALYSE DES RESULTATS

Les résultats obtenus pour chaque groupe clinique sont analysés par confrontation et opposition directe. Différents schémas d'analyses statistiques sont utilisés : courbes montrant le nombre de vrais positifs (sensibilité) en fonction du nombre de faux positifs (1 - spécificité) (ROC - *Receiver Operating Characteristic),* analyse par composante principale (ACP), analyse par classification (CART - *Classification And Regression* Tree), corrélations, etc... L'ensemble, testé et validé, assure la robustesse des modèles prédictifs construits.

## C - ETUDES EFFECTUEES

### C - 1 Introduction

Plusieurs études ont été menées (E1, E2, E3, E4, E5, E6), ayant pour objectif d'optimiser la visualisation des protéines dans les zones spectrales expliquées précédemment, d'optimiser leur détection et l'analyse de leur comportement en fonction de leurs propriétés biochimiques intrinsèques et de leurs capacités d'interaction et d'association. Plusieurs conditions ont été définies en fonction des propriétés chimiques des surfaces ou des polymères, des tampons d'incubation, du pH, des matrices dont la fonction est de ioniser les espèces et de faciliter leur désorption, donc leur séparation sous l'impulsion du laser (voir Tableau 2 plus haut décrivant les conditions utilisées).
Les caractéristiques principales de l'étude E6 sont :

o Etude menée en aveugle
o Etude rétrospective/Cas Contrôle
o Stockage des échantillons à l'Hôpital: -20°C & -80°C
o Stockage des échantillons à Neurolab: -80°C

Trois populations ont été étudiées :

o Contrôle = Groupe témoin
o DS = *Down Syndrome* = T21
o ES = *Edward Syndrome* = T18
o Une quatrième population, constituée d'échantillons congelés et conservés à -80°C renforce la valeur des pics analysés.

| Tableau 5 : codes | |
|---|---|
| Contrôle | 9, 10, 12, 13, 14, 15, 16, A9, A10, A11, A12, A13, A14, A15, A16 |
| DS | 1, 2, 3, 4, 5, 6, 7, 8, A1, A2, A3, A4, A5, A6, A7, A8 |
| ES | E3,E4,E5,E6 |
| -80°C | A25, A26, A27, A28 |

Remarques : Les annotations de la population étudiée et les résultats du Triple Test (hCG, uE3, $\alpha$ féto protéine) (examen de référence) montrent que les patientes $\Lambda$1 et 3 n'ont pas été dépistées, les enfants sont nés atteints du Syndrome de Down (Trisomie 21).

Les échantillons sont appariés (Contrôle/DS) pour éviter les biais de confusion.

En raison de l'excellente reproductibilité des expériences menées dans les études E1 à E5 en triplicate, un spectre est enregistré par échantillon, sauf pour contrôler la reproductibilité intra-expérience (par exemple : les échantillons A7 et A15 qui ont été enregistrés en triplicate lors de l'étude E6).

Les propriétés de la population d'étude sont résumées dans le Tableau 6 suivant et présentent les codifications suivantes:

DS = *Down Syndrome* = T21
ES = *Edward Syndrome* = T18
DM = Donnée Manquante à la Date du Rapport
N/A = Non Applicable (prélèvements effectués en dehors de la période étudiée)
IMG = Interruption Médicale de Grossesse
PV = perdu de vue. Une partie des données est donc manquante.

| Tableau 6 : Propriétés et Caractéristiques de la Population Etudiée (étude E6) | | | | | | | | | | | |
|---|---|---|---|---|---|---|---|---|---|---|---|
| Etat | Code | Age Maternel | Aménorrhée Sem + Jour | hCG | uE3 | AFP | Ris que | Resultats TT | Caryotype | Sexe | Issue |
| Contrôle | 9 | 33 | 16+1 | 1.55 | 0.87 | 0.35 | 1/90 | Faux + | Normal | M | Né |
| Contrôle | 10 | 30 | 15+3 | 1.58 | 0.89 | 0.99 | 1/970 | - | NA | M | Né |
| Contrôle | 11 | 24 | 15+4 | 1.50 | 0.53 | 0.93 | 1/175 | Faux + | Normal | M | Né |
| Contrôle | 12 | 28 | 15+0 | 1.22 | 0.94 | 0.80 | 1/1900 | - | NA | M | Né |
| Contrôle | 13 | 26 | 15+4 | 2.37 | 0.63 | 1.03 | 1/116 | Faux + | Normal | F | Né |
| Contrôle | 14 | 28 | 16+0 | 2.24 | 0.72 | 0.66 | 1/95 | Faux + | Normal | F | Né |
| Contrôle | 15 | 22 | 17+1 | 1.76 | 0.74 | 0.54 | 1/182 | Faux + | Normal | F | Né |
| Contrôle | 16 | 33 | 17+1 | 1.68 | 0.94 | 0.87 | 1/510 | - | Normal | F | Né |
| Contrôle | A9 | 33 | 16+4 | 1.46 | 0.88 | 0.74 | 1/332 | - | NA | F | Né |
| Contrôle | A10 | 33 | 16+3 | 1-20 | 0.63 | 0.92 | 1/474 | - | NA | PV | PV |
| Contrôle | A11 | 24 | 16+3 | 1.51 | 0.59 | 0.94 | 1/254 | Faux + | Normal | F | PV |
| Contrôle | A12 | 27 | 15+2 | 1.34 | 0.32 | 1.06 | 1/431 | - | Normal | M | Né |
| Contrôle | A13 | 36 | 14+6 | 1.97 | 0.45 | 0.86 | 1/11 | Faux + | Normal | F | Né |
| Contrôle | A14 | 34 | 15+2 | 1.36 | 0.6 | 0.7 | 1/76 | Faux + | Normal | M | Né |
| Contrôle | A15 | 38 | 17+5 | 1.80 | 0.94 | 1.37 | 11239 | Faux + | Normal | F | Né |
| Contrôle | A16 | 38 | 15+2 | 1.87 | 0.93 | 1.53 | 1/166 | Faux + | Normal | M | Né |
| | | | | | | | | | | | |
| Malade | 1 | 35 | 16+1 | 4.02 | 0.70 | 0.42 | 1/10 | + | DS | M | IMG |
| Malade | 2 | 27 | 15+5 | 4.02 | 0.42 | 1.07 | 1/8 | + | OS | M | IMG |
| Malade | 3 | 25 | 15+3 | 2.68 | 1.00 | 0-70 | 1/560 | Faux - | DS | M | Né |
| Malade | 4 | 32 | 15+0 | 2.22 | 1.26 | 0.77 | 11660 | Faux - | DS | M | IMG |
| Malade | 5 | 31 | 15+4 | 2.22 | 0.62 | 1.32 | 1/126 | + | DS | F | IMG |
| Malade | 6 | 29 | 15+6 | 2.41 | 0.62 | 1.06 | 1/84 | + | DS | F | IMG |
| Malade | 7 | 36 | 17+1 | 3.99 | 0.46 | 1.12 | 1/5 | + | DS | F | IMG |

| Tableau 6 : Propriétés et Caractéristiques de la Population Etudiée (étude E6) | | | | | | | | | | | |
|---|---|---|---|---|---|---|---|---|---|---|---|
| Etat | Code | Age Maternel | AménorrhéeSem + Jour | hCG | uE3 | AFP | Ris que | Resultats TT | Caryotype | Sexe | Issue |
| Malade | 8 | 21 | 16+4 | 2.57 | 0.60 | 1.28 | 1/143 | + | DS | F | IMG |
| Malade | A1 | 28 | 16+3 | 1.61 | 0.82 | 0.71 | 1/441 | Faux - | DS | F | Né |
| Malade | A2 | 36 | 16+4 | 1.11 | 0.66 | 0.69 | 1/153 | + | DS | M | Né |
| Malade | A3 | 30 | 15+4 | 3.46 | 0.52 | 1.33 | 1/17 | + | DS | F | IMG |
| Malade | A4 | 31 | 16+4 | 1.49 | 0.31 | 0.56 | 1/37 | + | DS | M | IMG |
| Malade | A5 | 34 | 16+3 | 3.87 | 0.63 | 0.48 | 1/5 | + | DS | F | IMG |
| Malade | A6 | 30 | 16 | 2.39 | 0.55 | 0.36 | 1/10 | + | DS | M | IMG |
| Malade | A7 | 38 | 17+2 | 1.29 | 1.26 | 0.61 | 1/310 | Faux - | DS | F | IMG |
| Malade | A8 | 40 | 17 | 1.84 | 0.76 | 0.81 | 1/32 | + | DS | M | IMG |
| | | | | | | | | | | | |
| Malade | E3 | 30 | 16 | 0.97 | 0.43 | 0.56 | 1/57 | + | ES | M | IMG |
| Malade | E4 | 35 | 16+1 | 0.54 | 0.21 | 1.33 | 1/9400 | Faux - | ES | F | IMG |
| Malade | E5 | 37 | 16+5 | 0.14 | 0.44 | DM | 1/2700 | Faux - | ES | M | IMG |
| Malade | E6 | 37 | 15+2 | 0.42 | 0.64 | 0.54 | 1/590 | Faux - | ES | M | IMG |
| | | | | | | | | | | | |
| Malade | A25 | 35 | 17+5 | 3.79 | 0.69 | 0.64 | 1/6 | + | DS | M | IMG |
| Malade | A26 | 34 | 15+1 | 4.91 | 0.99 | 0.83 | 1/22 | + | DS | F | IMG |
| Malade | A27 | 35 | 23+4 | 2.64 | 0.98 | 1.22 | 1/184 | N/A | DS M | | IMG |
| Malade | A28 | 34 | 20 | 3.95 | 1.50 | 1.61 | 1/920 | N/A | DS | F | IMG |

Dans cette application particulière (conditions retenues documentées), les échantillons ont été soit analysés natifs non fractionnés, soit analysés fractionnés en fonction de la masse des protéines. Les éluats ont été utilisés soit natifs soit analysés après déplétion en albumine ou autres protéines de transport, puis séparés et purifiés selon deux modes, l'un anionique, l'autre hydrophobe. Un analyseur de type spectromètre de masse SELDI-TOF a été utilisé pour acquérir les informations spectrales.

Intérêt, Associativité et Purification sur surface anionique

Les spectres présentés sont obtenus sur surface séparative de type anionique. Les pics d'intérêt mentionnés sont présents à pH 4 et à toute valeur du pH supérieure à 4, dans la mesure où aucun phénomène d'agrégation ou de précipitation des protéines n'intervient à pH basique.

Intérêt, Associativité sur surface hydrophobe

Les spectres présentés sont obtenus sur surface séparative de type hydrophobe, après phase d'incubation et de lavage en tampon *Phosphate Buffer Saline* (PBS 1X) à pH 7,4 (Sambrook, J., Fritsch, E.F. & Maniatis, T. (1989). *Molecular Cloning : A laboratory manual,* Cold Spring Harbor Laboratory Press, Cold Spring Harbor, N.Y.).

**C - 2 Schéma Expérimental (études E1, E2, E3, E4 et E6)**

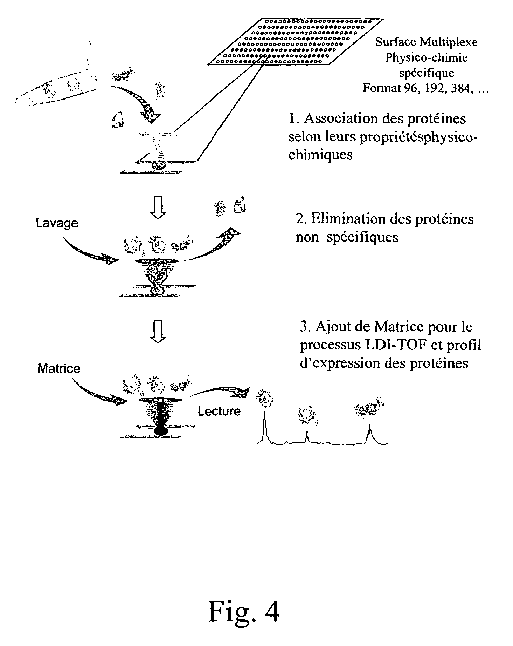

La Figure 4 présente le schéma expérimental qui a été utilisé.

Lors d'études de type profilage, les inventeurs ont travaillé en conditions non dénaturantes comme en conditions dénaturantes. Des puces à protéines ont été préparées selon le mode opératoire suivant :

1. Insertion des barrettes dans les bioprocesseurs ; 2. Hydratation ou pré rinçage des spots avec 5 à 100 $\mu$l d'un tampon d'hydratation spécifique ; 3. Élimination du surnageant et Ajout de 100 $\mu$l d'un tampon d'incubation ; 4. Ajout de 1 $\mu$l de sérum par spot ; 5. Incubation 10 minutes à 1h45, 6. Elimination du surnageant ; 7. Lavage de la surface avec 100 $\mu$l d'un tampon de lavage spécifique ; 8. Elimination du surnageant ; 9. Ajout de la matrice (CHCA ou SPA) ; 10. Séchage.

Les puces à protéines sont insérées dans le spectromètre de masse ou tout autre appareil de lecture ou de séquençage approprié.

Les différentes conditions séparatives et de purification utilisées et leurs protocoles de préparation sont récapitulés dans le Tableau 2 ci-dessus.

Lors d'une autre étude en approche ciblée (de type phosphorylation ou utilisant un anticorps) (étude E2), les inventeurs ont préparé des puces à protéines avec des échantillons biologiques natifs non fractionnés ou fractionnés, selon le mode opératoire suivant :

1. Insertion des barrettes dans les bioprocesseurs ; 2. Hydratation ou pré rinçage des spots avec 20 $\mu$l du tampon d'hydratation spécifique, 2 fois 5 minutes ; 3. Association des molécules de capture ; 4. Elimination du surnageant et Ajout de 100 $\mu$l d'un tampon d'incubation, 3 fois 5 minutes ; 5. ajout de 1 $\mu$l de sérum sur chaque spot ; 6. Incubation 30 minutes ; 7. Elimination du surnageant ; 8. Lavage de la surface avec 3 fois 100 $\mu$l, 5 minutes, d'un tampon de lavage spécifique ; 9. Elimination du surnageant et lavage final avec 100 $\mu$l d'eau pure ; 10. Ajout de la matrice (CHCA ou SPA diluée au 1/10); 11. Séchage.

Les puces à protéines sont insérées dans le spectromètre de masse pour lecture selon les différentes intensités décrites plus haut et à la Fig. 3, ou dans tout autre appareil de lecture approprié, optimisé et réglé en conséquence. L'homme du métier choisira l'intensité du laser pour permettre de faire voler les protéines, en fonction de leur masse, de certaines de leurs propriétés physico-chimiques, et d'éventuelles interactions qu'elles établissent avec des partenaires. Les anticorps utilisés ont été décrits plus haut.

Les calibrants utilisés sur puce Phase Normale/Oxyde de silicium et en mode Maldi correspondent à ceux décrits dans le Tableau 3.

Lors de ces différentes études, la séparation avec fractionnement a été réalisée, générant les quatre fractions suivantes :

Fraction A : poids moléculaire $\leq$ 10 kDa
Fraction B : 10 kDa < poids moléculaire $\leq$ 50 kDa
Fraction C : 50 kDa < poids moléculaire $\leq$ 100 kDa

Fraction D : poids moléculaire > 100 kDa

Le fractionnement en fonction de la masse à pour but d'augmenter la concentration relative de protéines d'une fraction par rapport à la concentration en protéines totales, sans pour autant modifier la concentration absolue des protéines séparées. Dans la fraction C, l'albumine est normalement retrouvée comme élément majoritaire, et peut être, le cas échéant, spécifiquement supprimée. Cette suppression n'a pas été effectuée dans les expériences décrites ici ; la suppression de l'albumine permettra de détecter un pic supplémentaire, correspondant à l'$\alpha$-fétoprotéine, qui est pour l'instant masquée par le pic d'albumine.

Ces fractions ont été obtenues en utilisant des séparateurs de type Vivaspin 500 de la société Vivascience, avec 3 seuils de coupure distincts : 10, 50 et 100 kDa. La séparation par filtration sur membrane a lieu en PBS 1X, pH 7,4, en conditions non dénaturantes. Tout ce qui passe au travers de la membrane est de plus petite masse que le seuil de coupure, tout ce qui reste dans le volume mort au-dessus de la membrane est de masse supérieure au seuil de coupure. Les protéines restant dans la partie supérieure du Vivaspin 500 avec un seuil de coupure de 100 kDa ont donc une masse native > à 100 kDa. Cela s'entend également pour les complexes multimériques hétérogènes aussi bien que homogènes.

**C - 3 Instrumentation (Exemple : Etude E6 - Purification anionique)**

Paramètres d'acquisition

Système d'acquisition : *Proteinchip Biomarker System* Type II.c

| Tableau 7 : Paramètres généraux d'acquisition de l'Etude Principale (E6) | |
|---|---|
| Protocole d'enregistrement | Paramètres d'acquisition |
| 1. Régler la masse élevée à 50000 Daltons, optimisée de 2500 Daltons à 15000 Dallons<br>2. Régler l'intensité laser de départ à 170.<br>3. Régler la sensibilité du détecteur à 10.<br>4. Régler la masse du déflecteur à 2500 Daltons. | Instrument:<br><br>Type: PBS IIc<br>Serial Num: 2F359TC |
| Statistiques d'enregistrement | Acquisition : |
| Tirs :<br><br>Effectués : 2814; Conservés : 2680 ; Haut : 0 ; Bas : 0<br>Intensité:<br><br>Basse: 170; Haute : 175<br>Sensibilité:<br><br>Basse : 10 ; Haute : 10 | Fréquence du numériseur : 500.0 MHz<br>Mode ionique : Positif<br><br>Voltage de la source: 20000 V<br>Voltage du détecteur: 2900 V<br>Voltage de l'impulsion : 3000 V<br>Durée de l'impulsion: 700 ns<br>Focus de la masse : 8750.00 Da |
| Période d'enregistrement | Mode déflecteur : Manuel |
| Etude E6 | Masse du déflecteur : 2500 Da |

| Tableau 8 : Paramètres spécifiques d'acquisition de l'Etude Principale (E6) | | | |
|---|---|---|---|
| Echantillons | Vide | Echantillons | Vide |
| A1 | 1.935e-007 Torr | 1 | 2.072e-007 Torr |
| A2 | 1.673e-007 Torr | 2 | 1.798e-007 Torr |
| A3 | 1.575e-007 Torr | 3 | 1.673e-007 Torr |
| A4 | 1.575e-007 Torr | 4 | 1.575e-007 Torr |
| A5 | 2.072e-007 Torr | 5 | 1.673e-007 Torr |

Suite de tableau

| Tableau 8 : Paramètres spécifiques d'acquisition de l'Etude Principale (E6) | | | |
|---|---|---|---|
| Echantillons | Vide | Echantillons | Vide |
| A6 | 1.798e-007 Torr | 6 | 1.575e-007 Torr |
| A7 (1) | 1.673e-007 Torr | 7 | 1.477e-007 Torr |
| A7 (2) | 1.380e-007 Torr | 8 | 1.380e-007 Torr |
| A7 (3) | 1.380e-007 Torr | 9 | 1.935e-007 Torr |
| A8 | 1.575e-007 Torr | 10 | 1.798e-007 Torr |
| A9 | 1.798e-007 Torr | 11 | 1.673e-007 Torr |
| A10 | 1.673e-007 Torr | 12 | 1.575e-007 Torr |
| A11 | 1.575e-007 Torr | 13 | 1.575e-007 Torr |
| A12 | 1.477e-007 Torr | 14 | 1.477e-007 Torr |
| A13 | 1-935e-007 Torr | 15 | 1.380e-007 Torr |
| A14 | 1.935e-007 Torr | 16 | 1.380e-007 Torr |
| A15 (1) | 1.380e-007 Torr | E3 | 1.282e-007 Torr |
| A15 (2) | 1.673e-007 Torr | E4 | 1.282e-007 Torr |
| A15 (3) | 1.673e-007 Torr | E5 | 1.575e-007 Torr |
| A16 | 1.282e-007 Torr | E6 | 1-477e-007 Torr |

Rapport de Normalisation des Spectres

Le procédé de normalisation "*Total* Ion Current" (TIC) permet pour chaque spectre de faire la moyenne des intensités, et ajuste les intensités moyennes de chaque spectre de sorte que les données soient normalisées.
Les paramètres de normalisation utilisés correspondaient à une normalisation *"Total Ion Current"* commençant à 3000, et se terminant à 20000, avec soustraction de la ligne de base, un coefficient de normalisation de 0.269112, et sans normalisation par rapport à la masse.

| Tableau 9 : Rapport de Normalisation (Etude Principale - Séparation Anionique) | | | | | | | |
|---|---|---|---|---|---|---|---|
| *Nom de l'échantillon* | *Facteur de normalisation* | *Utilisé en normalisation?* | *Avec TIC* | *Nom de l'échantillon* | *Facteur de normalisation* | *Utilisé en normalisation?* | *Avec TIC* |
| A01 | 1,255852 | oui | 0,214286 | 1 | 2,450139 | oui | 0,109835 |
| A02 | 1,664225 | oui | 0,161704 | 2 | 0,808214 | oui | 0,332971 |
| A03 | 2,185387 | oui | 0,123142 | 3 | 1,07021 | oui | 0,251457 |
| A04 | 1,291102 | oui | 0,208436 | 4 | 2,306647 | oui | 0,116668 |
| A05 | 0,974679 | oui | 0,276103 | 5 | 0,743689 | oui | 0,361861 |
| A06 | 0,648508 | oui | 0,414971 | 6 | 0,974983 | oui | 0,276017 |
| A07 | 0,560603 | oui | 0,48004 | 7 | 2,918434 | oui | 0,092211 |
| A07 | 1,307443 | oui | 0,205831 | 8 | 0,719411 | oui | 0,374073 |
| A07 | 1,895244 | oui | 0,141993 | 9 | 0,975285 | oui | 0,275932 |
| A08 | 0,821502 | oui | 0,327585 | 10 | 1,17547 | oui | 0,22894 |
| A09 | 1,261377 | oui | 0,213348 | 11 | 2,192259 | oui | 0,122756 |
| A10 | 0,751794 | oui | 0,35796 | 12 | 1,508624 | oui | 0,178382 |
| A11 | 1,233307 | oui | 0,218204 | 13 | 1,387028 | oui | 0194021 |
| A12 | 1,249535 | oui | 0,21537 | 14 | 1,690433 | oui | 0,159197 |
| A13 | 1,068304 | oui | 0,251906 | 15 | 1,568808 | oui | 0,171539 |
| A14 | 0,600652 | oui | 0,448034 | 16 | 1,648534 | oui | 0,163243 |
| A15 | 1,233057 | oui | 0,218248 | E3 | 1,89569 | oui | 0,14196 |
| A15 | 1,263684 | oui | 0,212958 | E4 | 1,437991 | oui | 0,187144 |
| A15 | 3,701159 | oui | 0,07271 | E5 | 1,241774 | oui | 0,216716 |
| A16 | 1,244716 | oui | 0,216204 | E6 | 2,593202 | oui | 0,103776 |

Rapport de Calibration

La calibration est faite en fonction de la gamme de masses observée. Dans la zone 3 à 20 kDa, trois calibrations sont utilisées, selon que le calibrant est :

- Insuline bovine
- Cytochrome C, bovin
- β-Lactoglobuline-A, bovine

Par exemple entre 2.9 et 5.8 kDa, la calibration est faite sur les pics d'insuline (bovine) (5733.58+1H), et d'insuline bovine 2H+ (2866.79+1H).

*Equation de calibration*

L'équation de calibration utilisée est m/Z quadratique :

$$\frac{m/z}{U} = a(t - t_0)^2 + b$$

où m/z est le rapport masse sur charge, U le voltage de la source et t le temps de vol.

*Paramètres d'acquisition (Exemple : Cas de l'insuline)*

Deux conditions d'enregistrement des spectres, pour deux concentrations en insuline bovine, ont été utilisées.

| Tableau 10 : conditions d'enregistrement | |
|---|---|
| Protocole d'enregistrement : Protocole 1, INSULINE Bovine | Paramètres d'acquisition |
| 1. Régler la masse élevée à 50000 Daltons, optimisée de 1000 Daltons à 10000 Daltons.<br>2. Régler l'intensité laser de départ à 120.<br>3. Régler la sensibilité du détecteur à 10.<br>4. Régler la masse du déflecteur à "auto". | Instrument :<br><br>Type: PBS lic<br>Serial Num 2F359TC |
| Statistiques d'enregistrement | Acquisition : |
| Tirs :<br><br>Effectués : 682 ; Conservés : 620 ; Haut : 0 ; Bas : 0<br><br>Intensité<br><br>Basse : 120 ; Haute : 130<br><br>Sensibilité:<br><br>Basse : 10 ; Haute :10 | Fréquence du numériseur : 500.0 MHz<br><br>Mode ionique : Positif<br><br>Voltage de la source: 20000 V<br>Voltage du détecteur: 2900 V<br>Voltage de l'impulsion : 3000 V<br>Durée de l'impulsion: 564 ns<br>Focus de la masse : 5500.00 Da |
| Période d'enregistrement | Mode déflecteur: Auto |
| Etude E6 | Masse du déflecteur : 1000 Da |

*Résultats de l'acquisition après calibration (Cas de l'insuline)*

| Tableau 11 | | | | |
|---|---|---|---|---|
| Paramètres | Valeurs | Paramètres | Valeurs | |
| $a$ | $0.358981048675136$ $\times 10^{-3}$ | $t_0$ | $1.11897791939812$ | $\dfrac{m/z}{U} = a(t - t_0)^2 + b$ |
| $b$ | $8.2752735372077$ $\times 10^{-3}$ | $U$ | $20000\ V$ | |

Après calibration, l'écart par rapport théorique est égal à 0.00 Dalton (*i.e.*, précision supérieure à deux ordres de grandeur).

Rapport de clusterisation

*Définition*

Afin de comparer le niveau d'expression d'une protéine selon les échantillons, la clusterisation consiste à regrouper la valeur de l'intensité des pics correspondants à cette protéine pour l'ensemble des spectres enregistrés. Un *cluster* se définit donc comme un tableau retournant la valeur de l'intensité du pic considéré pour chaque spectre étudié.

*Clusterisation*

Trois groupes sont clusterisés simultanément pour pouvoir faire un comparatif ultérieur :
Contrôles (*Control*), DS (*Down Syndrome*) et ES (*Edward Syndrome*).
Les annotations ont été réalisées selon deux modes complémentaires, l'un manuel, par les expérimentateurs, l'autre automatique.

*Mode de clusterisation manuel*

Les pics sont manuellement annotés.
Les clusters sont ensuite complétés par recherche dans une fenêtre de $\pm$ 0,3% de la valeur M+H d'un signal tel que signal/bruit = 2, sinon, le bruit de fond local est utilisé.

*Mode de clusterisation automatique*

La détection des pics obéit à 2 critères :

    o le rapport signal sur bruit doit être $\geq$ 10
    o le pic doit être présent dans 30 % des spectres.

Les clusters sont ensuite complétés par recherche dans une fenêtre de $\pm$ 0,3 % de la valeur M+H d'un signal tel que signal/bruit = 2, sinon, le bruit de fond local est utilisé.

*Conclusion sur la zone Spectrale visualisée dans l'exemple choisi*

Compte tenu de la qualité spectrale (bruit de fond, qualité du signal et du rapport signal/bruit) :

- la zone de visualisation est comprise entre 3 000 Da et 20 000 Da
- la zone d'analyse est comprise entre 4 000 Da et 20 000 Da

La calibration est faite sur les pics d'Insuline (bovine) (5733.58+1H), et d'insuline bovine 2H+ (2866.79+1H). L'objectif est d'optimiser la zone de calibration comprise entre 2800 et 5750 Da. Entre 6 et 12 kDa, le cytochrome C (bovin) est utilisé pour calibrer. Entre 9 et 19 kDa, la β-lactoglobuline A (bovin) est utilisée pour calibrer.
Le choix du calibrant n'influence en aucune façon les valeurs des intensités. Leurs valeurs ne varient pas en fonction de la calibration utilisée. La calibration permet d'accroître la précision des valeurs des masses des pics enregistrés, sur une zone spectrale restreinte.

Outre les calibrants mentionnés plus haut, d'autres calibrants peuvent être utilisés. Le Tableau 12 ci-après présente une liste de calibrants qui peuvent être utilisés, ainsi que leur poids moléculaire.

| Tableau 12 | |
|---|---|
| Peptide / protéine | Poids moléculaire (Daltons) |
| Arg8-Vasopressine | 1084,2474 |
| Angiotensine I humaine | 1296,5 |
| [Glu[1]] Fibrinopeptide B | 1570,6 |
| Somatatostatine | 1637,9030 |
| Dynorphine A (209-225) porcine | 2147,5 |
| ACTH (1-24) humaine | 2933,5 |
| β-endorphine humaine | 3465,0 |
| Chaîne β de l'insuline bovine | 3495,9409 |
| insuline bovine | 5733,6 |
| Insuline humaine | 5807,6533 |
| Ubiquitine bovine | 8564,8 |
| Cytochrome C bovin | 12230,9 |
| Cytochrome C équin | 12360,2 |
| Superoxide dismutase bovine | 15591,4 |
| Myoglobine équine | 16951,1 ou 16951,5* |
| β-lactoglobuline A bovine | 18363,3 |
| GAPDH de lapin | 35688,0 |
| Péroxidase de raifort | 43240,0 |
| Sérum albumine bovine (BSA) | 66410,0 ou 66433,0* |
| Hirudine BHVK | 7033,6136 |
| Conalbumine de poulet | 77490,0 |
| β-galactosidase de *E.coli* | 116351,0 |
| IgG bovine | 147300,0 |
| * : indication de poids moléculaire différente suivant les fournisseurs. | |

## D - RESULTATS

### D - 1 Synthèse des Résultats Analytiques obtenus

```
        Résultats  Analytiques  =  Résolution  Spectrale
    en Masse et en Intensité
```

Les inventeurs ont identifié des clusters d'intérêt (référencés dans les Tableaux 13 et 14) dans les données issues des spectres obtenus (un spectre par échantillon des populations des trois groupes Contrôle, DS (T21) et ES (T18), hors groupe -80°C, sauf cas particulier des échantillons A7 et A15, enregistrés en triplicate lors de l'étude E6). Le nombre de clusters annotés manuellement est réduit selon le critère "présent dans au moins 30 % de la population spectrale".

| Tableau 13 : Synthèse Phase Analytique | | | | | | |
|---|---|---|---|---|---|---|
| Expériences | | Zone d'analyse | Mode de Clusterisation | Nombre Clusters identifiés | Nombre de clusters retenus | Nombre Spectres Analysés |
| Phase séparative anionique | E6 | 4-20 kDa | Manuel | 156 | 29 (18.6 %) | 40 |
| | | | Automatique | 8 | 7 (87.5%) | |
| | E1-E5 | 3-10 kDa | Manuel | 40 | 39 (97.5 %) | 32 |
| | | | Automatique | 6 | 6(100%) | |
| Interaction hydrophobique | E6 | 3-10 kDa | Manuel | 140 | 31 (22.2 %) | 34 |
| | | | Automatique | 17 | 17 (100 %) | |
| | | 3-20 kDa | Manuel | 164 | 54 (32.9 %) | 34 |
| | | | Automatique | 35 | 34 (97.2 %) | |

| Tableau 14 : Liste des clusters retenus | | | | |
|---|---|---|---|---|
| CLUSTERS | Phase séparative anionique | | Interaction hydrophobique E6 | Présence d'un signal sur des échantillons congelés à -80°C |
| | E6 | Cumul E1-E5 | | |
| PM3061 | | | +* | - |
| PM3101 | | | + | + |
| PM3156 | | | + | + |
| PM3166 | | | +* | + |
| PM3181 | | | +* | + |
| PM3214 | | | + | + |
| PM3223 | | | + | + |
| PM3233 | | | +* | + |
| PM3313 | | | + | + |
| PM3320 | + | - | | + |
| PM3321 | | | + | - |
| PM3371# | + | + | | + |
| PM3394 | | | +* | - |
| PM3400 | + | - | | + |
| PM3442# | + | + | | + |
| PM3486# | + | + | | + |
| PM3504 | | | +* | + |
| PM3678 | | | + | + |

Suite de tableau

| CLUSTERS | Phase séparative anionique | | Interaction hydrophobique E6 | Présence d'un signal sur des échantillons congelés à -80°C |
|---|---|---|---|---|
| | E6 | Cumul E1-E5 | | |
| PM3705 | | | + | + |
| PM3815 | + | - | | ++ |
| PM3846 | + | - | | ++ |
| PM4059 | | | + | + |
| PM4067 | | | + | + |
| PM4110 | + | - | | + |
| PM4111 | | | + | + |
| PM4152# | + | + | | + |
| PM4168# | + | + | | + |
| PM4186 | + | + | | + |
| PM4197 | | | + | |
| PM4201# | + | + | | + |
| PM4217# | - | + | | + |
| PM4279 | | | +* | + |
| PM4286 | | | + | + |
| PM4295 | | | + | + |
| PM4300# | + | + | | + |
| PM4317 | | | + | + |
| PM4332 | + | + | | + |
| PM4349 | | | + | + |
| PM4410 | | | + | + |
| PM4412 | - | + | | + |
| PM4463 | | | + | + |
| PM4470 | | | + | + |
| PM4530 | - | + | | + |
| PM4583 | | | +* | + |
| PM4650 | | | + | + |
| PM4682# | | | +* | - |
| PM4706 | | | +* | + |
| PM4722 | | | +* | + |
| PM4816 | | | + - | + |
| PM4821 | + | + | | + |
| PM4860 | - | + | | + |
| PM4893 | - | + | | + |
| PM4935 | - | + | | + |

Tableau 14 : Liste des clusters retenus

Suite de tableau

| Tableau 14 : Liste des clusters retenus | | | | |
|---|---|---|---|---|
| CLUSTERS | Phase séparative anionique | | Interaction hydrophobique E6 | Présence d'un signal sur des échantillons congelés à -80°C |
| | E6 | Cumul E1-E5 | | |
| PM5008 | - | + | | + |
| PM5033 | | | + | + |
| PM5222 | - | + | | + |
| PM5625# | | | + | + |
| PM5630 | - | + | | + |
| PM5695# | + | + | | + |
| PM5712 | - | + | | + |
| PM5720 | | | +* | - |
| PM5756 | | | + | + |
| PM5793 | - | + | | + |
| PM5799 | | | + | + |
| PM5982 | - | + | | + |
| PM6222# | + | + | | + |
| PM6299 | + | + | | + |
| PM6423# | | | + | + |
| PM6430 | - | + | | + |
| PM6439# | | | + | + |
| PM6461 | | | + | + |
| PM6470 | - | + | | + |
| PM6482 | | | + | + |
| PM6548 | - | + | | + |
| PM6605 | | | +* | + |
| PM6620# | | | | + |
| PM6624 | - | + | | + |
| PM6636# | | | + | + |
| PM6659 | | | + | + |
| PM6679 | | | + | + |
| PM6764 | | | +* | + |
| PM6782 | | | + | - |
| PM6808 | - | + | | + |
| PM6937 | + | + | | + |
| PM7019 | - | + | | + |
| PM7020 | | | + | + |
| PM7652 | - | + | | + |
| PM8112 | | | + | + |

Suite de tableau

| | Phase séparative anionique | | Interaction hydrophobique E6 | Présence d'un signal sur des échantillons congelés à -80°C |
|---|---|---|---|---|
| **Tableau 14 : Liste des clusters retenus** | | | | |
| CLUSTERS | E6 | Cumul E1-E5 | | |
| PM8176 | + | + | | + |
| PM8206 | + | + | | + |
| PM8220 | | | + | - |
| PM8226 | - | + | | + |
| PM8558 | - | + | | + |
| PM8563 | | | + | + |
| PM8581 | | | +* | - |
| PM8594 | + | + | | + |
| PM8627 | | | + | + |
| PM8691 | | | + | + |
| PM8695 | | | | + |
| PM8717 | | | +* | + |
| PM8809 | | | + | + |
| PM8872 | | | +* | + |
| PM8917 | | | +* | + |
| PM8929 | | | + | + |
| PM9291 | | | + | + |
| PM9358 | + | + | | + |
| PM9360# | | | + | + |
| PM9401 | | | +* | - |
| PM9407 | | | +* | + |
| PM9617 | | | +* | - |
| PM10051 | | | + | - |
| PM11093 | | | +* | - |
| PM11369 | + | N/A | | + |
| PM12434 | + | N/A | | + |
| PM12575 | + | N/A | | + |
| PM12862 | | | + | - |
| PM13313 | + | N/A | | + |
| PM13316 | | | + | + |
| PM14027 | + | N/A | | + |
| PM14035# | | | + | + |
| PM14430 | | | + | - |
| PM16672 | + | N/A | | + |
| PM16995.56 | + | N/A | | |

Suite de tableau

| Tableau 14 : Liste des clusters retenus | | | | |
|---|---|---|---|---|
| CLUSTERS | Phase séparative anionique | | Interaction hydrophobique E6 | Présence d'un signal sur des échantillons congelés à -80°C |
| | E6 | Cumul E1-E5 | | |
| PM17262# | | | + | + |
| PM17348.75 | + | N/A | | + |
| PM17386 | | | + | + |
| PM17581.18 | + | NIA | | + |
| PM18901.94 | + | N/A | | + |
| PM19139.2 | + | N/A | | + |
| PM19558.27 | + | N/A | | + |
| PM19624.73 | + | N/A | | + |
| PM19845.94 | + | N/A | | + |
| Légende du Tableau 14 :<br>+* : Critère de sélection des clusters moins stringent appliqué à la phase séparative par interactions hydrophobiques :<br>-> [% présent Groupe Contrôle] + [% présent Groupe T21] >= 30<br>++ : Cluster présentant un signal sur des échantillons stockés à -80°C, 10 fois plus important qu'avec des échantillons stockés à -20°C.<br># : Clusters présentés ultérieurement à titre d'exemple<br>- : pas d'observation | | | | |

Les clusters définis au Tableau 14 correspondent aux marqueurs de la liste (i) ci-dessus.

**D - 2 Qualité des données et observations**

Plusieurs critères qualitatifs permettent de juger de la qualité des données. On peut citer, à titre d'exemples, la reproductibilité des spectres obtenus pour un même échantillon, les écarts de temps de vol (proportionnels à la masse des protéines analysées) observés pour un même cluster, les paramètres de normalisation des spectres, ou encore la valeur du rapport signal sur bruit, les coefficients de variation souvent très inférieurs à 10% etc...

Le Tableau 15 montre un exemple de bonne reproductibilité de la valeur du TOF (donc de la masse réelle observée) et de la qualité du rapport signal sur bruit sur les Etudes E1 à E6.

Le Tableau 16, présenté également à titre d'exemple, indique pour quelques clusters observés lors des expériences de l'étude E6, le temps de vol (TOF), la valeur p du cluster et pour chaque groupe (Contrôle, T21, T18), les intensités moyenne, minimale et maximale ainsi que la déviation standard sur l'intensité.

**Tableau 15 : Expérience Q10 cumul E1 à E5 / Mode d'annotation automatique**
**TABLEAU PARTIEL PRESENTE A TITRE D'EXEMPLE**

| Définition du cluster | | | | | Validité du cluster | | | | | | Qualité du signal | | | | | | | |
| --- | --- | --- | --- | --- | --- | --- | --- | --- | --- | --- | --- | --- | --- | --- | --- | --- | --- | --- |
| | | | | | Représentation du cluster | Temps de vol | | | | | Rapport Signal sur Bruit | | | | | | | |
| Cluster | Masse Moyenne des substances (Calibration selon le cytochrome C bovin) | Mode d'annotation | Numéro du cluster | Calibrant | % annotations manuelles | % pics détectés lors de la clusterisation | TOF (µs) | Deviation (ns) | Minimum (µs) | Maximum (µs) | Minimum | 25% percentile | Médiane | 75% percentile | Maximum | Moyenne | Déviation standard | Erreur standard |
| PM4152 | 4151,00 / 4163,88 | Automatique | 1 | Insuline Bovine (50% de la population spectrale) / Cytochrome C Bovin | N/A | 100.00 | 24,67 | 16,93 | 24,65 | 24,69 | 2,50 | 20,87 | 38,47 | 67,56 | 179,62 | 55,92 | 52,20 | 9,23 |
| PM4168 | 4166,38 / 4179,04 | | 2 | | N/A | 72.00 | 24,71 | 16,91 | 24,69 | 24,74 | 1,17 | 5,82 | 15,69 | 24,07 | 43,79 | 15,74 | 10,99 | 1,94 |
| PM4186 | 4184,41 / 4196,54 | | 3 | | N/A | 66.67 | 24,76 | 18,29 | 24,74 | 24,79 | 0,78 | 3,67 | 13,60 | 32,66 | 112,80 | 20,69 | 24,02 | 4,25 |
| PM4300 | 4300,03 / 4311,45 | | 4 | | N/A | 100.00 | 25,10 | 16,63 | 25,08 | 25,13 | 4,34 | 7,92 | 13,19 | 19,40 | 26,75 | 13,63 | 6,48 | 1,15 |
| PM4821 | 4817,60 / 4823,26 | | 5 | | N/A | 100.00 | 26,56 | 17,11 | 26,54 | 26,59 | 7,06 | 15,08 | 18,00 | 25,15 | 35,94 | 19,66 | 7,13 | 1,26 |
| PM8594 | 8590,93 | | 6 | Cytochrome C Bovin | N/A | 85.71 | 35,44 | 16,95 | 35,42 | 35,47 | 1,04 | 4,39 | 6,44 | 11,81 | 25,56 | 8,27 | 5,02 | 0,89 |

30

| Tableau 16 : Etude principale E6 / Séparation hydrophobique / Rapport sur les Intensités / Annotations Automatiques / Normalisation selon le clusterPM6620 **TABLEAU PARTIEL PRESENTE A TITRE D'EXEMPLE** | | | | | | | | | | | | | | | |
|---|---|---|---|---|---|---|---|---|---|---|---|---|---|---|---|
| Cluster | N° Cluster | TOF (µs) | Valeur p | Contrôle | | | | T21 | | | | T18 | | | |
| | | | | Moyenne | Standard déviation | Min | Max | Moyenne | Déviation standard | Min | Max | Moyenne | Déviation standard | Min | Max |
| PM3156 | 1 | 21.54 | 0.457208 | 4.45 | 4.12 | -0.46 | 13.73 | 5.90 | 5.95 | 1.04 | 22.23 | 2.56 | 1.68 | 0.99 | 4.49 |
| PM3215 | 2 | 21.73 | 0.009008 | 8.24 | 1.68 | 5.80 | 13.67 | 10.16 | 2.05 | 6.88 | 13.96 | 9.18 | 3.17 | 5.24 | 12.84 |
| PM4410 | 11 | 25.43 | 0.295773 | 5.68 | 5.58 | 2.56 | 27.60 | 5.37 | 3.09 | 2.63 | 14.80 | 4.41 | 1.47 | 3.16 | 6.53 |
| PM4463 | 12 | 25.59 | 0.179243 | 8.32 | 4.96 | 1.82 | 18.69 | 8.94 | 4.70 | 2.58 | 19.42 | 7.48 | 4.73 | 3.13 | 12.41 |
| PM4470 | 13 | 25.61 | 0.0982 | 9.37 | 4.13 | 3.04 | 16.95 | 10.98 | 6.06 | 4.37 | 29.35 | 9.05 | 3.32 | 5.07 | 11.99 |
| PM4650 | 14 | 26.11 | 0.103567 | 6.08 | 5.20 | 1.17 | 21.43 | 8.89 | 16.22 | 1.42 | 70.95 | 6.10 | 3.09 | 2.67 | 9.92 |
| PM5033 | 15 | 27.16 | 0.006125 | 2.99 | 2.11 | 0.71 | 8.97 | 4.47 | 8.59 | 0.86 | 38.29 | 3.49 | 1.47 | 2.04 | 5.53 |
| PM5625 | 16 | 28.70 | 0.044947 | 3.38 | 3.96 | 0.51 | 16.82 | 1.76 | 0.90 | 0.46 | 3.70 | 0.98 | 0.36 | 0.60 | 1.34 |
| PM5756 | 17 | 29.04 | 0.005933 | 8.41 | 3.26 | 4.09 | 16.07 | 10.91 | 7.23 | 2.71 | 33.55 | 8.96 | 6.19 | 5.05 | 18.13 |
| PM5799 | 18 | 29.15 | 0.010931 | 10.11 | 4.50 | 4.86 | 20.92 | 12.99 | 8.19 | 3.96 | 38.00 | 9.28 | 5.33 | 5.10 | 17.04 |
| PM6423 | 19 | 30.68 | 0.004728 | 13.35 | 2.05 | 9.74 | 19.41 | 16.51 | 2.88 | 12.15 | 24.46 | 14.32 | 4.01 | 9.41 | 19.21 |
| PM6439 | 20 | 30.72 | 0.188758 | 14.73 | 3.46 | 8.00 | 20.73 | 21.24 | 15.24 | 9.88 | 78.67 | 17.08 | 6.40 | 9.78 | 25.13 |
| PM6620 | 21 | 31.14 | 0.288472 | 28.31 | 0.07 | 28.24 | 28.57 | 28.29 | 0.03 | 28.23 | 28.38 | 28.29 | 0.01 | 28.27 | 28.30 |
| PM6636 | 22 | 31.18 | 0.552805 | 23.09 | 4.62 | 16.10 | 35.12 | 24.67 | 7.12 | 15.34 | 47.82 | 28.92 | 6.58 | 23.60 | 38.16 |
| PM6659 | 23 | 31.23 | 0.024619 | 7.35 | 1.08 | 5.52 | 9.41 | 8.82 | 2.31 | 5.71 | 15.44 | 9.75 | 1.38 | 8.64 | 11.76 |
| PM7020 | 24 | 32.07 | 0.063323 | 6.00 | 4.30 | 1.58 | 19.43 | 4.21 | 1.97 | 1.32 | 7.88 | 2.43 | 0.46 | 2.03 | 2.91 |
| PM8112 | 25 | 34.47 | 0.073538 | 1.52 | 1.12 | 0.05 | 3.45 | 3.64 | 7.49 | 0.14 | 32.68 | 2.27 | 2.33 | 0.05 | 4.44 |
| PM8563 | 26 | 35.41 | 0.012477 | 9.92 | 9.13 | 4.20 | 44.52 | 11.84 | 8.61 | 5.18 | 41.52 | 10.54 | 6.65 | 5.48 | 19.92 |
| PM8627 | 27 | 35.54 | 0.006125 | 16.10 | 5.60 | 8.25 | 30.85 | 22.41 | 14.89 | 10.09 | 73.99 | 17.95 | 11.28 | 10.78 | 34.71 |
| PM8691 | 28 | 35.67 | 0.012396 | 23.91 | 8.73 | 12.95 | 43.53 | 32.12 | 20.38 | 13.57 | 99.68 | 22.72 | 12.74 | 13.67 | 41.61 |
| PM8809 | 29 | 35.91 | 0.115651 | 8.15 | 3.41 | 5.16 | 20.22 | 10.57 | 6.68 | 5.19 | 33.76 | 7.45 | 3.03 | 4.82 | 11.81 |
| PM8929 | 30 | 36.15 | 0.010007 | 7.83 | 2.32 | 4.69 | 12.39 | 10.67 | 6.37 | 5.86 | 32.45 | 7.77 | 1.84 | 5.02 | 8.88 |
| PM9291 | 31 | 36.87 | 0.325234 | 4.34 | 2.26 | 1.75 | 11.24 | 6.11 | 8.93 | 1.70 | 40.62 | 3.89 | 1.59 | 2.18 | 5.70 |
| PM9360 | 32 | 37.01 | 0.170855 | 8.94 | 5.65 | 2.31 | 25.13 | 6.76 | 3.71 | 2.20 | 18.58 | 4.08 | 0.21 | 3.90 | 4.38 |
| PM14035 | 33 | 45.28 | 0.175758 | 4.79 | 3.58 | 0.67 | 14.43 | 3.04 | 1.71 | 0.62 | 6.05 | 1.61 | 0.35 | 1.14 | 1.97 |
| PM17262 | 34 | 50.19 | 0.008505 | 4.80 | 1.57 | 2.22 | 8.55 | 6.94 | 4.57 | 2.97 | 23.46 | 5.07 | 3.03 | 2.97 | 9.57 |
| PM17386 | 35 | 50.37 | 0.020274 | 5.77 | 2.17 | 2.71 | 10.50 | 8.12 | 5.11 | 3.28 | 26.12 | 5.41 | 2.98 | 3.40 | 9.83 |

**D - 3 Spectres et Figures**

Profils obtenus par séparation et purification de type anionique Q10 - étude E6:

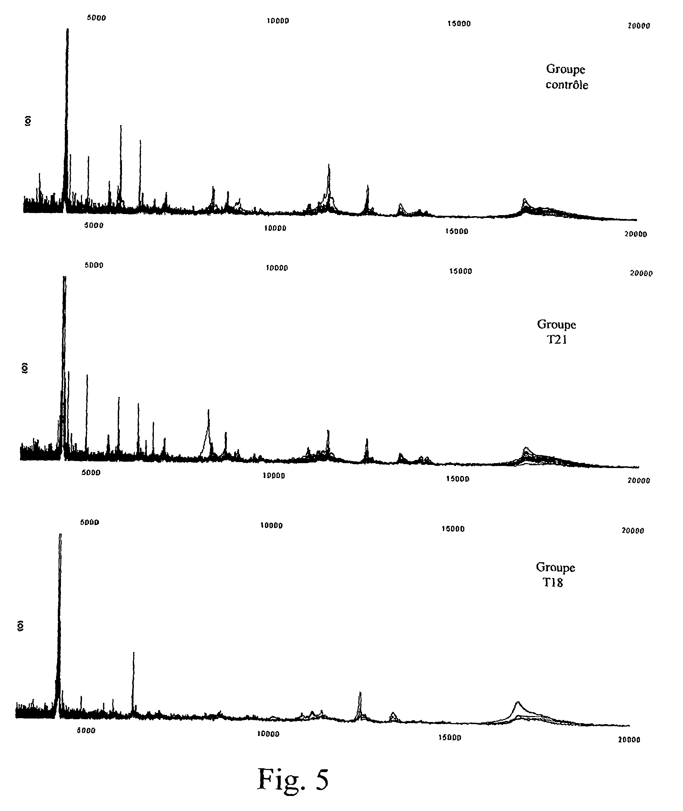

La figure 5 montre les spectres obtenus sur la zone spectrale 3000-20000 Daltons, en vues globales, pour le groupe contrôle, le groupe T21 et le groupe T18. Les spectres normalisés y sont superposés par catégorie clinique.

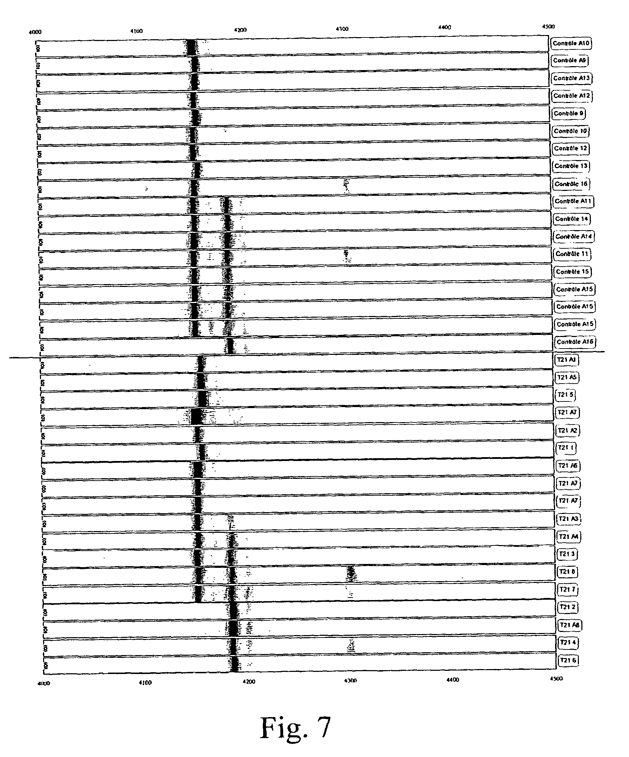

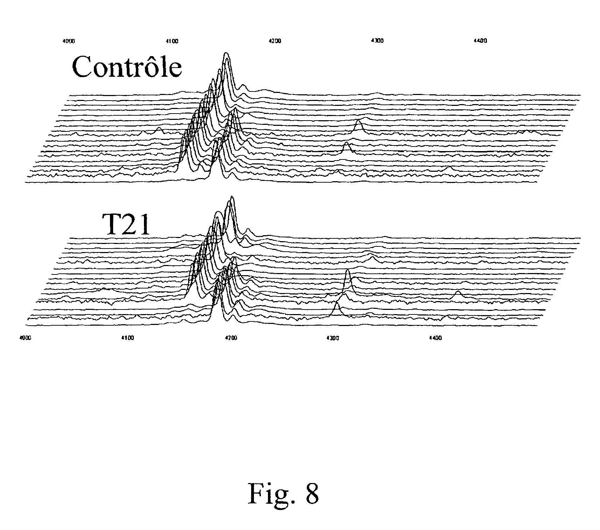

Les figures 6, 7 et 8 montrent, respectivement, les vues spectrales (spectres normalisés et superposés par catégorie clinique), les vues en pseudo-gels, et les spectres en vues décalées (étude E6), centrés sur la zone 4000 Da.

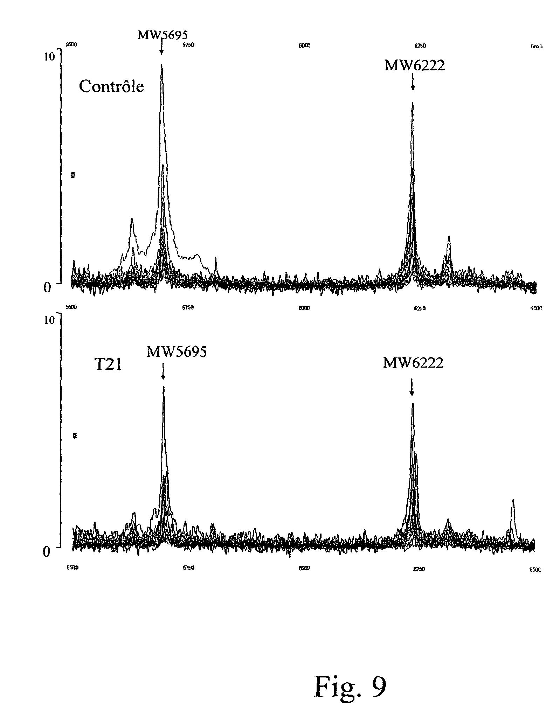

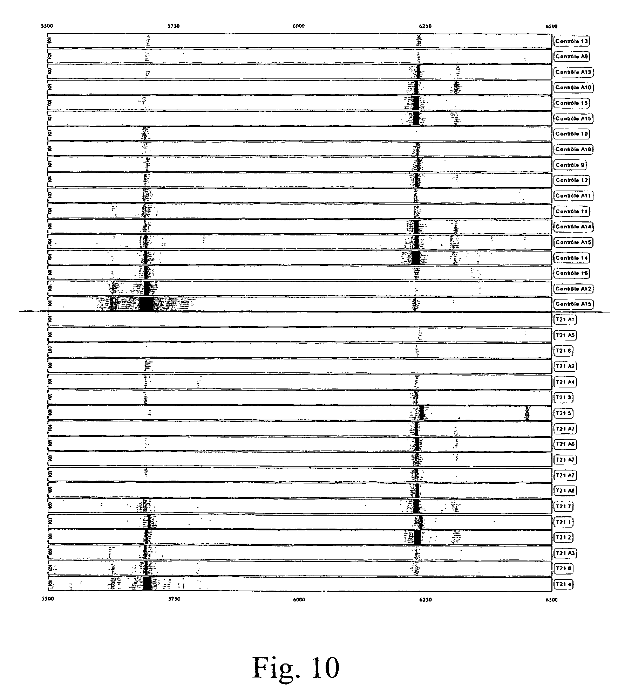

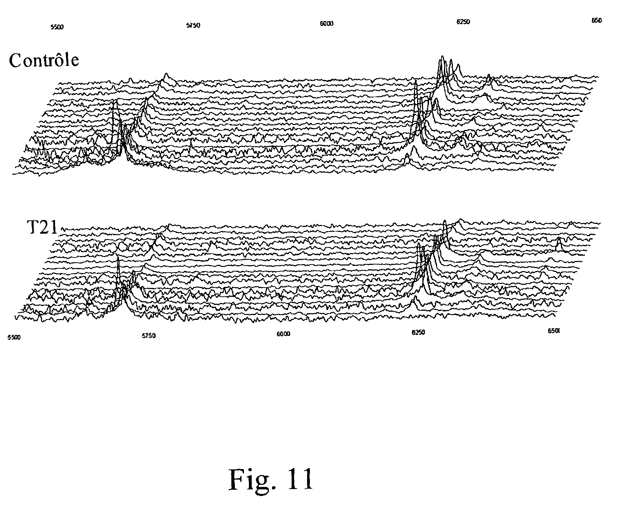

Les figures 9, 10 et 11 montrent, respectivement, les vues spectrales (spectres normalisés et superposés par catégorie clinique), les vues en pseudo-gels, et les spectres en vues décalées (étude E6), centrés sur la zone 5000-6000 Da.

Profils obtenus par séparation de type anionique Q10 : E1 à E5

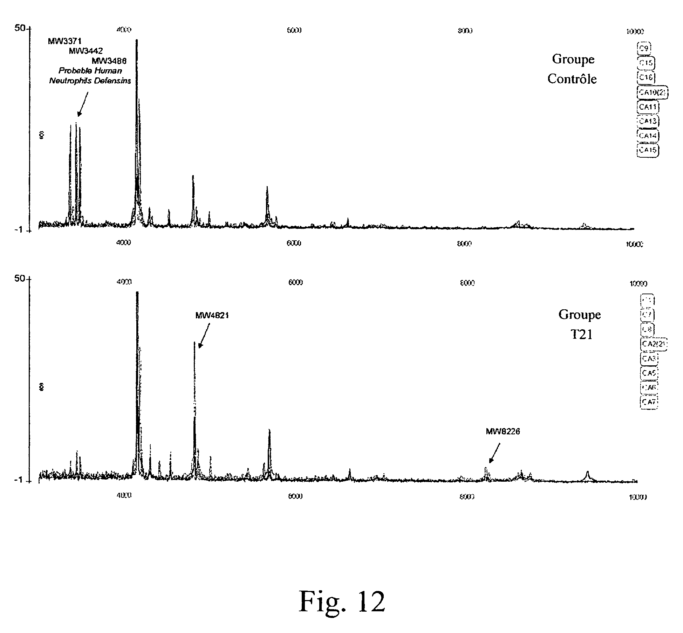

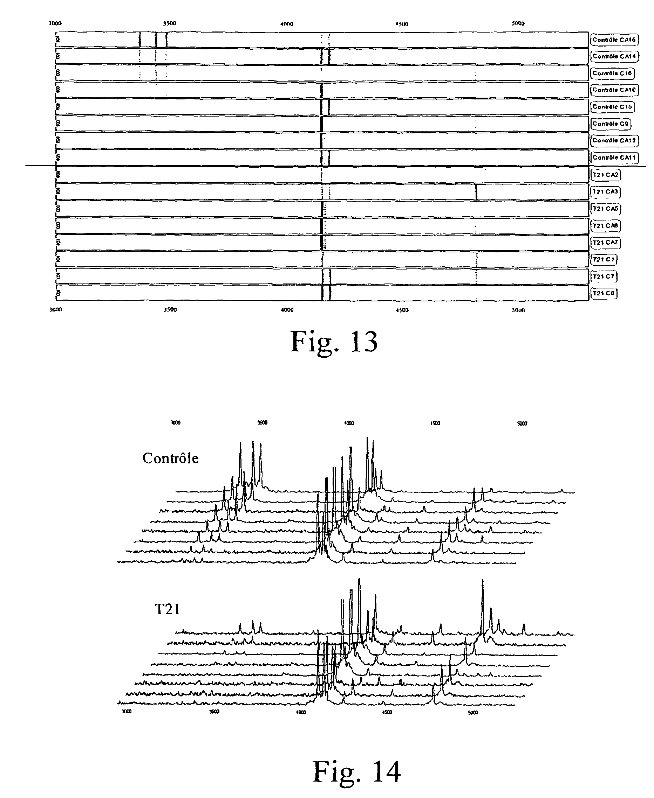

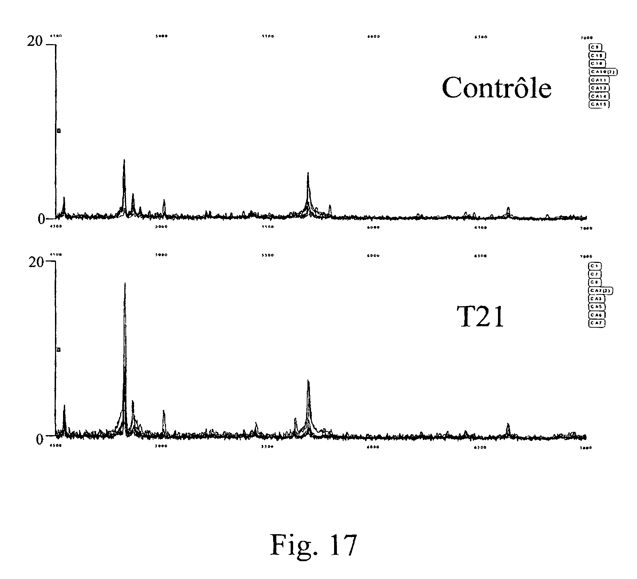

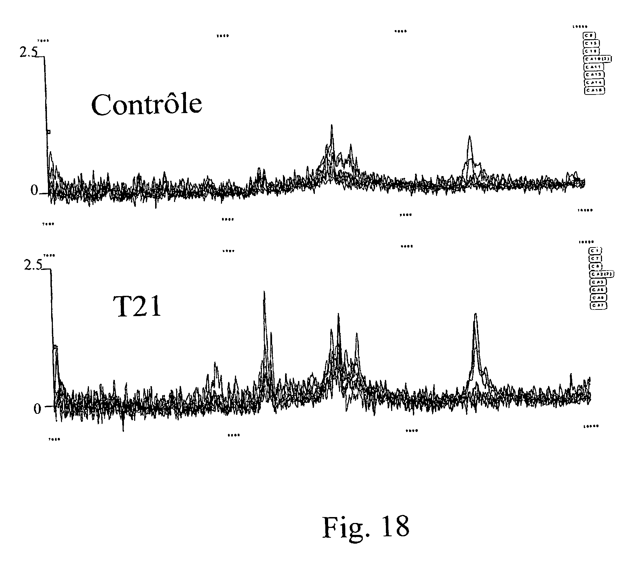

Ces profils sont montrés sur les figures 12 à 18, pour les deux catégories cliniques (contrôles et T21).

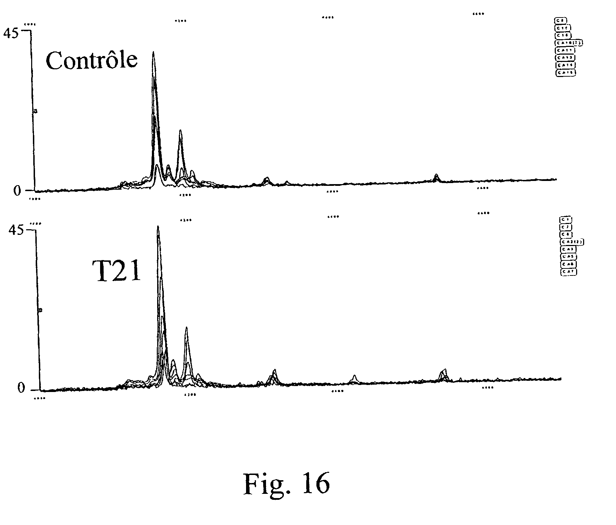

Les figures 12 à 14 montrent les spectres centrés sur la zone spectrale 3000-10000 Daltons, respectivement en vues globales (spectres normalisés et superposés par catégorie clinique), vue pseudo-gel, et vues décalées (E1 à E5). La figure 15 montre les spectres centrés sur la zone spectrale 3000-4000 Daltons (E1 à E5). Les figures 16 à 18 montrent les spectres, normalisés et superposés par catégorie clinique, centrés respectivement sur les zones 4000-5000 Daltons, 5000-7000 Daltons, et 7000-10000 Daltons (études E1 à E5).

Profils obtenus par séparation selon l'hydrophobicité des protéines H50 (étude E6)

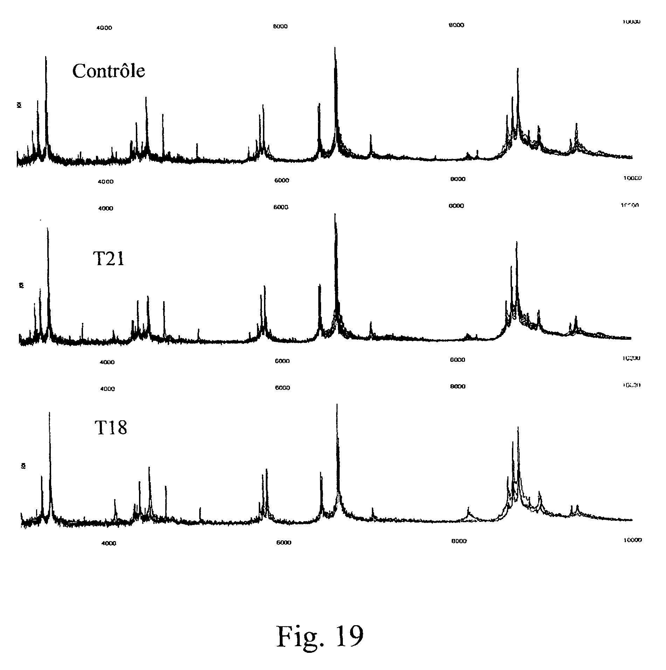

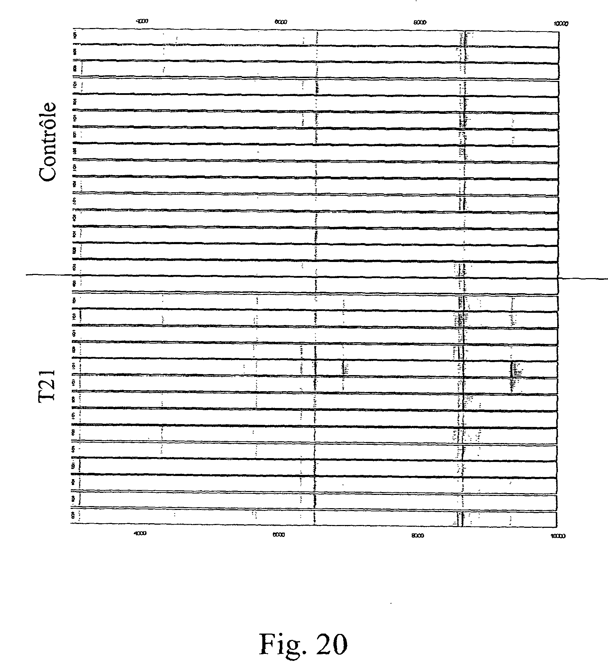

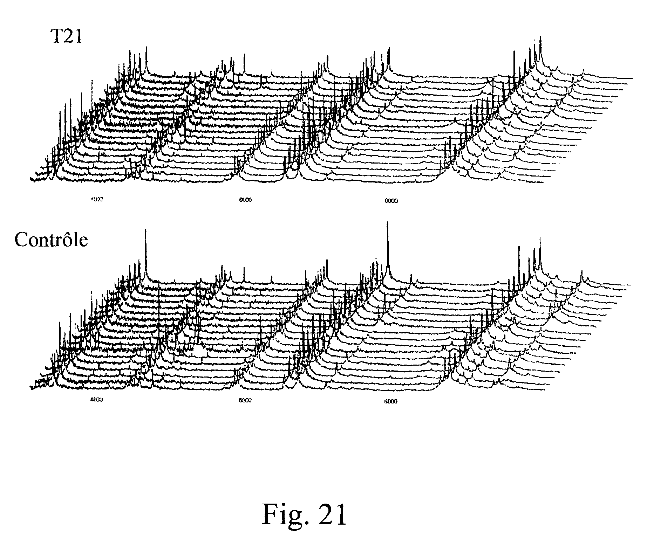

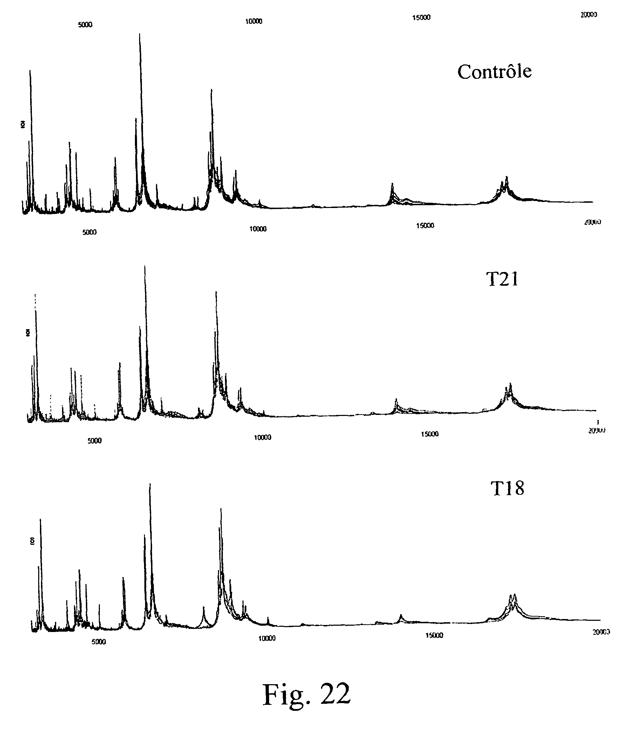

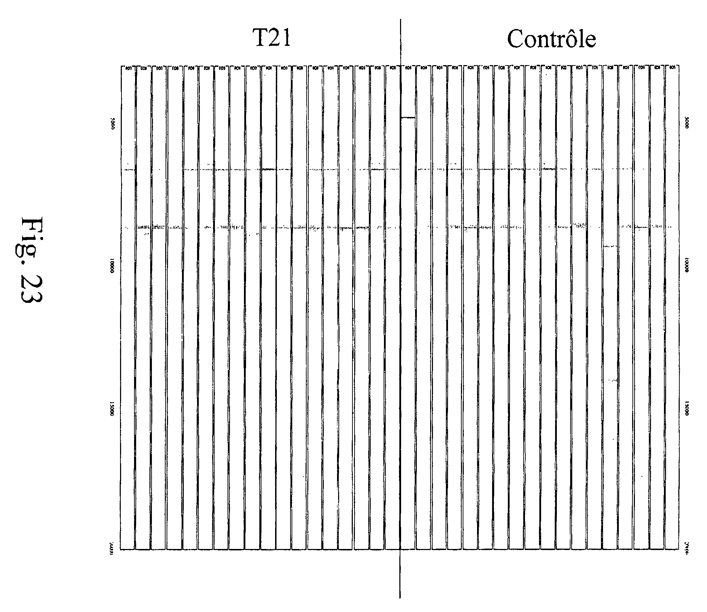

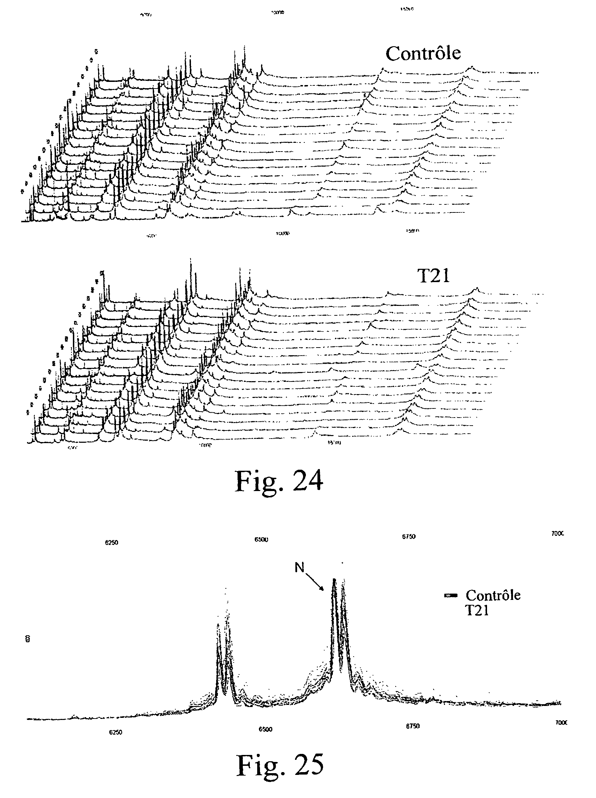

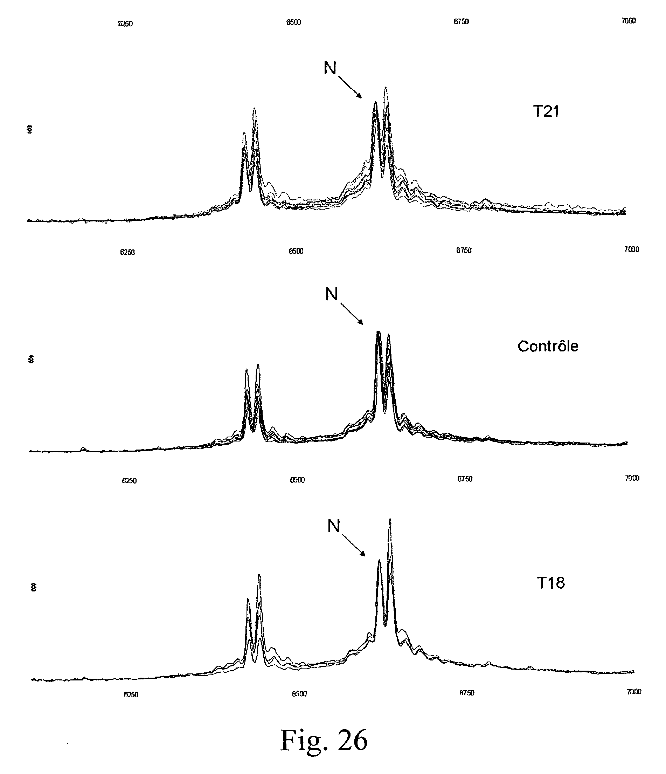

Ces profils sont présentés dans les figures 19 à 26.

**D - 4 Exemples de statistiques de distribution des intensités dans les groupes**

Les figures 27 à 30 montrent des exemples de statistiques de distributions des intensités de différents clusters, pour les groupes contrôle et T21.

**D - 5 Exemples de Courbes ROC construites à partir** des Aires sous la courbe des Intensités brutes

Les aires sous la courbe ROC *(Receiver Operating Characteristic)* des pics d'intérêts, calculées à partir des intensités brutes, sont plus grandes que 0,50, et peuvent atteindre 0,95.

La figure 31 montre les courbes ROC pour les marqueurs de poids moléculaires 3371, 3442, 3486, et 6423 Da.

**D - 6 Exemple de Classification Hiérarchique sur Dataset E6 et Anionique**

La figure 32 montre le résultat d'une construction avec les logiciels Genecluster Version 1999 (Eisen M.) et Treeview Version 1.60 (Eisen M.) (Classification hiérarchique sur Jeu de Données de l'étude E6 (IC 95%), Surface Anionique Q10).

Méthode de construction : Ajustement des données par transformation logarithmique puis normalisation dans l'ordre, selon les spectres puis les clusters. Puis clusterisation hiérarchique simultanément sur les clusters et les spectres par corrélation de Spearman.

Résultats : Excellente discrimination des Groupes contrôle ("bleu") , T21 ("rouge"), Délétion 7q (Série C), T18 (série E), Série T21 -80°C ("noire"), Série Contrôle -80°C ("verte").

**D - 7 Exemple d'Analyse en Composante Principale sur Datasets des études E2 à E5 et Anionique**

Les logiciels utilisés sont le logiciel Stata SE 8 et le logiciel *Ciphergen Express* Version 1.0. Les deux logiciels confirment les résultats.

Les valeurs propres (ou Valeurs de Eigen) confirment la présence et le poids moléculaire des pics identifiés comme étant des défensines dont les masses sont : PM3371, PM3442 et PM3486.

Les résultats sont montrés sur la figure 33.

**D - 8 Résultats cliniques et applications pour le dépistage**

Les résultats obtenus pour les pics dans la zone 3000 Daltons, sur surface anionique Q10, sont présentés dans le Tableau 17. Ces trois pics sont sous-exprimés ou absents dans le groupe T21, traduisant une baisse importante de l'immunité, et exprimés normalement ou sur-exprimés dans le groupe contrôle. Une étude plus approfondie des résultats permettra de déterminer le seuil de détection pour ces trois pics, qui sont corrélés et dépendants, tel qu'au moins un signe négatif parmi les trois ou un signe positif parmi les trois signe la maladie ou l'absence de maladie, respectivement. Les résultats présentés dans le Tableau 17 présentent une variabilité pour ces trois pics, principalement liée au fait qu'ils ont été obtenus avec des échantillons conservés à -20°C, présentant une certaine dégradation.

Le Tableau 18 ci-après présente les résultats et applications pour les pics dans la zone 4000 - 9000 Daltons, sur surface anionique Q10.

Le Tableau 19 montre les résultats et applications pour les pics dans la zone 4000 -17500 Daltons, sur surface hydrophobe H50.

| Tableau 17 : Sélection des échantillons / Phase séparative anionique, pH > 4 / Clusters PM3371, PM3442 et PM3486 | | | | |
|---|---|---|---|---|
| Echantillons | Groupe | Q10 cumul E1 - E5 | | |
| | | PM3371 | PM3442 | PM3486 |
| 1 | DS | - | - | - |
| 2 | DS | - | - | - |
| 4 | DS | - | - | + |
| 5 | DS | - | - | - |
| 6 | DS | - | - | + |
| 7 | DS | - | - | - |
| 8 | DS | - | - | - |
| A1 | DS | - | - | - |
| A2 | DS | - | + | + |
| A2 | DS | + | + | + |
| A3 | DS | - | - | + |
| A4 | DS | + | + | - |
| A5 | DS | - | - | - |
| A6 | DS | - | - | - |
| A7 | DS | - | - | - |
| A8 | DS | - | - | - |
| 9 | Contrôle | + | + | + |
| 10 | Contrôle | - | + | + |
| 12 | Contrôle | + | + | + |
| 13 | Contrôle | + | - | + |
| 14 | Contrôle | + | + | + |
| 15 | Contrôle | + | + | + |
| 16 | Contrôle | + | + | + |
| A9 | Contrôle | - | - | - |
| A10 | Contrôle | + | + | + |
| A10 | Contrôle | + | + | + |
| A11 | Contrôle | - | - | - |
| A12 | Contrôle | - | - | - |
| A13 | Contrôle | + | + | - |
| A14 | Contrôle | + | + | + |
| A15 | Contrôle | + | + | + |
| A16 | Contrôle | - | - | + |
| Aire sous la courbe ROC | | 0,8164 | 0,8555 | 0,8164 |
| Erreur Standard | | 0.07572 | 0,06688 | 0,07419 |
| Intervalle de confiance (95%) | | 0,6680 à 0,9649 | 0,7244 à 0,9866 | 0,6710 à 0,9619 |
| Valeur P | | 0,002279 | 0,0006092 | 0,002279 |

| Tableau 18 : Sélection des échantillons par clusters / Phase séparative anionique, pH > 4 / Quelques exemples | | | | | | | | | | | | | | |
|---|---|---|---|---|---|---|---|---|---|---|---|---|---|---|
| Echan-tillons | Groupes | Q10 cumul E1 à E5 | | | | | | | Etude principale E6 | | | | | | |
| | | PM4152 | PM4168 | PM4186 | PM4201 | PM4300 | PM5695 | PM6222 | PM4152 | PM4168 | PM4186 | PM4201 | PM4300 | PM5695 | PM6222 |
| 1 | DS | - | - | - | - | - | + | + | + | - | + | - | + | + | + |
| 2 | DS | - | - | + | + | + | - | + | + | - | + | + | + | - | + |
| 3 | DS | N/A | N/A | N/A | N/A | N/A | N/A | N/A | + | + | + | + | + | + | - |
| 4 | DS | - | - | + | + | + | + | + | - | - | + | - | + | - | + |
| 5 | DS | + | + | - | - | + | - | + | + | + | - | - | + | - | - |
| 6 | DS | - | - | + | + | + | + | - | + | - | + | + | - | - | - |
| 7 | DS | - | - | + | + | + | + | - | + | + | + | + | + | + | + |
| 8 | DS | + | + | + | + | - | + | - | + | + | + | + | + | + | + |
| A1 | DS | + | + | - | - | + | + | - | + | + | - | - | + | - | - |
| A2 | DS | - | - | - | - | + | - | - | + | + | - | - | + | - | - |
| A2 | DS | + | + | - | - | - | + | - | N/A | N/A | N/A | N/A | N/A | N/A | N/A |
| A3 | DS | - | - | + | + | + | + | - | + | + | + | + | + | - | - |
| A4 | DS | + | + | + | + | + | - | - | + | + | + | + | + | - | - |
| A5 | DS | + | + | - | - | + | + | - | + | + | + | + | + | - | + |
| A6 | DS | + | + | - | - | + | + | + | + | + | + | - | + | - | + |
| A7 | DS | + | + | - | - | - | + | + | + | + | + | - | + | - | + |
| A7 | DS | N/A | N/A | N/A | N/A | N/A | N/A | N/A | + | + | + | - | + | - | + |
| A7 | DS | N/A | N/A | N/A | N/A | N/A | N/A | + | + | - | + | + | - | - | + |
| A8 | DS | + | + | + | + | + | - | + | + | + | + | - | - | + | + |
| 9 | Contrôle | + | + | - | - | - | + | - | + | + | + | - | - | + | - |
| 10 | Contrôle | + | + | - | - | + | - | - | + | + | + | - | + | + | - |
| 11 | Contrôle | N/A | N/A | N/A | N/A | N/A | N/A | N/A | + | + | + | + | + | + | + |
| 12 | Contrôle | + | + | - | - | + | + | - | + | + | + | - | + | + | + |
| 13 | Contrôle | + | + | - | - | + | - | - | + | + | - | - | - | - | + |
| 14 | Contrôle | - | - | - | - | + | + | - | + | + | + | + | - | - | + |
| 15 | Contrôle | + | + | + | + | - | + | - | + | + | - | - | + | + | + |
| 16 | Contrôle | - | - | - | - | - | + | - | + | - | - | - | + | - | + |
| A9 | Contrôle | + | + | - | - | + | - | - | + | + | + | - | + | - | + |
| A10 | Contrôle | - | - | - | - | + | + | - | N/A | N/A | N/A | N/A | N/A | N/A | N/A |
| A10 | Contrôle | + | + | - | - | - | + | - | N/A | N/A | N/A | + | + | + | + |
| A11 | Contrôle | + | + | + | + | - | - | + | + | + | - | + | + | + | - |
| A12 | Contrôle | + | + | - | - | + | - | - | + | + | - | - | - | + | - |
| A13 | Contrôle | + | + | - | - | - | + | - | + | + | + | - | - | - | + |
| A14 | Contrôle | + | + | + | + | - | + | - | + | + | + | + | - | + | + |

34

| Tableau 18 : Sélection des échantillons par clusters / Phase séparative anionique, pH > 4 / Quelques exemples | | | | | | | | | | | | | | | |
|---|---|---|---|---|---|---|---|---|---|---|---|---|---|---|---|
| Echan-tillons | Groupes | Q10 cumul E1 à E5 | | | | | | | Etude principale E6 | | | | | | |
| | | PM4152 | PM4168 | PM4186 | PM4201 | PM4300 | PM5695 | PM6222 | PM4152 | PM4168 | PM4186 | PM4201 | PM4300 | PM5695 | PM6222 |
| A15 | Contrôle | - | - | + | + | - | + | + | + | + | + | + | - | - | + |
| A15 | Contrôle | N/A | N/A | N/A | N/A | N/A | N/A | N/A | + | + | + | + | - | + | + |
| A15 | Contrôle | N/A | N/A | N/A | N/A | N/A | N/A | N/A | + | + | + | + | + | + | + |
| A16 | Contrôle | - | - | + | + | + | - | + | + | - | + | + | - | + | + |
| A25 | Absent 80°C | N/A | N/A | N/A | N/A | N/A | N/A | N/A | N/A | N/A | Absent | Absent | Présent | Absent | Absent |
| A26 | Absent 80°C | N/A | N/A | N/A | N/A | N/A | N/A | N/A | Présent | Absent | Présent | Présent | Absent | Absent | Absent |
| A27 | Absent 80°C | N/A | N/A | N/A | N/A | N/A | N/A | N/A | Présent | Présent | Absent | Absent | Présent | Présent | Absent |
| A28 | Absent 80°C | N/A | N/A | N/A | N/A | N/A | N/A | N/A | Présent | Absent | Présent | Présent | Absent | Présent | Absent |
| Aire sous la courbe ROC | | 0,5820 | 0,6055 | 0,6055 | 0,6758 | 0,5781 | 0,5820 | 0,5781 | 0,5772 | 0,5401 | 0,5401 | 0,5648 | 0,6790 | 0,6543 | 0,6080 |
| Erreur Standard | | 0,1050 | 0,1029 | 0,1029 | 0,09707 | 0,1057 | 0,1042 | 0,1043 | 0,09804 | 0,1003 | 0,09832 | 0,09735 | 0,09221 | 0,09370 | 0,09568 |
| Intervalle de confiance (95%) | | 0.3761 to 0.7880 | 0.4037 to 0.8072 | 0.4037 to 0.8072 | 0.4855 to 0.8661 | 0.3710 to 0.7853 | 0.3777 to 0.7864 | 0.3736 to 0.7826 | 0.3850 to 0.7694 | 0.3435 to 0.7368 | 0.3474 to 0.7329 | 0.3740 to 0.7557 | 0.4982 to 0.8598 | 0.4706 to 0.8380 | 0.4204 to 0.7956 |
| Valeur P | | 0,4287 | 0,3089 | 0,3089 | 0,08995 | 0,4510 | 0,4287 | 0,4510 | 0,4290 | 0,6809 | 0,6809 | 0,5065 | 0,06656 | 0,1137 | 0,2682 |

| Tableau 19 : Sélection des échantillons par clusters / Phase séparative hydrophobique / Quelques exemples | | | | | | | | | | |
|---|---|---|---|---|---|---|---|---|---|---|
| Echantillons | Groupes | PM4682 | PM5625 | PM6423# | PM6439# | PM6620 | PM6636# | PM9360 | PM14035 | PM17262 |
| 1 | DS | + | - | - | + | - | + | - | - | + |
| 2 | DS | - | - | + | + | - | - | - | + | + |
| 3* | DS | + | - | + | + | - | + | + | - | + |
| 4 | DS | - | + | + | - | + | - | - | - | - |
| 5 | DS | - | - | + | - | + | - | - | - | + |
| 6 | DS | - | - | - | - | + | - | - | - | + |
| 7 | DS | - | - | + | + | - | + | + | - | + |
| 8 | DS | - | + | - | - | + | - | - | + | + |
| A1* | DS | - | - | + | + | - | + | - | + | - |
| A2 | DS | - | - | + | + | - | + | - | + | + |
| A3 | DS | - | - | + | - | + | - | - | + | + |
| A4* | DS | - | - | + | - | - | - | - | + | + |
| A5 | DS | - | - | + | + | + | - | - | - | + |
| A6 | DS | - | + | + | - | - | - | + | - | + |
| A7 | DS | - | - | + | - | - | + | - | - | - |
| A7 | DS | - | - | + | + | + | + | - | - | + |
| A7 | DS | - | - | + | + | + | + | + | - | + |
| A8 | DS | - | - | + | + | - | + | - | + | + |
| 9 | Contrôle | + | - | - | - | + | - | - | - | - |
| 10 | Contrôle | + | + | - | - | - | - | + | + | - |
| 11* | Contrôle | - | + | - | - | + | + | + | + | - |
| 12 | Contrôle | + | + | - | - | + | - | + | + | + |
| 13 | Contrôle | + | + | + | - | - | + | + | + | + |
| 14 | Contrôle | - | + | - | + | + | - | + | + | - |
| 15 | Contrôle | + | + | - | - | - | - | + | + | - |
| 16 | Contrôle | + | + | - | - | + | - | + | + | - |
| A9* | Contrôle | + | - | - | + | + | + | - | - | + |
| A10 | Contrôle | - | - | - | - | + | - | - | + | + |
| A11 | Contrôle | + | + | + | + | + | + | - | + | + |
| A12* | Contrôle | + | + | - | + | - | + | + | - | + |
| A13 | Contrôle | + | - | - | - | + | - | + | + | - |
| A14 | Contrôle | + | + | - | - | + | - | + | + | |

| Tableau 19 : Sélection des échantillons par clusters / Phase séparative hydrophobique / Quelques exemples | | | | | | | | | |
|---|---|---|---|---|---|---|---|---|---|
| Échantillons | Groupes | PM4682 | PM5625 | PM6423# | PM6439# | PM6620 | PM6636# | PM9360 | PM14035 | PM17262 |
| A15 | Contrôle | - | - | - | - | + | - | - | - | - |
| A15 | Contrôle | - | - | - | - | + | + | - | - | - |
| A15 | Contrôle | + | + | - | - | + | + | + | + | + |
| A16 | Contrôle | + | + | - | - | + | + | + | + | + |
| A25 | -80°C | Absent | Présent | Présent | Présent | Présent | Présent | Présent | Présent | Présent |
| A26 | -80°C | Absent | Présent | Présent | Présent | Présent | Présent | Présent | Présent | Présent |
| A27 | -80°C | Absent | Présent | Présent | Présent | Présent | Présent | Présent | Présent | Présent |
| A28 | -80°C | Absent | Présent | Présent | Présent | Présent | Présent | Présent | Présent | Présent |
| Aire sous la courbe ROC | | 0,8133 | 0,7111 | 0,6000 | 0,6265 | 0,7111 | 0,8622 | 0,6756 | 0,7600 | 0,5644 |
| Erreur Standard | | 0,08624 | 0,09526 | 0,1076 | 0,09633 | 0,09793 | 0,06877 | 0,09850 | 0,08770 | 0,1089 |
| Intervalle de confiance (95%) | | 0.6443 to 0.9824 | 0.5244 to 0.8979 | 0.3891 to 0.8109 | 0.4377 to 0.8154 | 0.5191 to 0.9031 | 0.7274 to 0.9970 | 0.4824 to 0.8687 | 0.5881 to 0.9319 | 0.3510 to 0.7779 |
| Valeur P | | 0,003469 | 0,04887 | 0,3507 | 0,1946 | 0,04887 | 0,0007292 | 0,1014 | 0,01528 | 0,5476 |

# : Critères sélectifs obtenus après normalisation des clusters par rapport à l'intensité du cluster PM6620

* : Echantillons utilisés exclusivement pour tester les critères de sélection

EP 1 624 074 A1

**D - 9 Purification et Identification : quelques exemples**

<u>Purification</u>

Parmi diverses combinaisons testées, surfaces de séparation et de purification, conditions de purification etc., on peut citer les suivantes :

Tableau 20 : Conditions de purification / Quelques exemples

| Conditions de séparation | 50 kDa < PM < 100 kDa | | | | | | PM > 100 kDa | | | | | | Sérum brut | |
|---|---|---|---|---|---|---|---|---|---|---|---|---|---|---|
| | Oxyde de silice | Hydrophobe pH et conditions physiologique C6-C18 | Approche ciblée Ion Gallium | Amine quaternaire pH4 | Amine quaternaire pH8 | Amine quaternaire pH et conditions physiologique | Oxyde de silice | Hydrophobe pH et conditions physiologique C6-C18 | Approche ciblée Ion Gallium | Amine quaternaire pH4 | Amine quaternaire pH8 | Amine quaternaire pH et conditions physiologique | hCG | PAPP-A |
| PM3371 | - | - | + | + | + | + | - | - | - | - | - | - | - | - |
| PM3442 | - | - | + | + | + | + | - | - | - | - | - | - | - | - |
| PM3486 | -* | - | + | + | + | + | - | - | - | - | - | - | - | - |
| PM6423 | -* | + | - | - | -* | -* | + | + | - | - | - | - | + | + |
| PM6439 | -* | + | - | - | -* | -* | + | + | - | - | - | - | + | + |
| PM6620 | -* | + | - | - | -* | -* | + | + | - | - | - | - | + | + |
| PM6636 | -* | + | - | - | -* | -* | + | + | - | - | - | - | + | + |

-* : Signal fortement perturbé par la présence massive d'albumine. Expériences après déplétion de l'albumine en cours.

Identification Clusters PM3371, PM3442 et PM3486 *Considérations sur la* masse *native*

Ces trois peptides ont des propriétés biophysicochimiques corrélées. Ils sont présents dans une fraction native des échantillons telle que la masse des produits soit comprise entre 50 et 100 kDa. Il s'agit donc de peptides associés à une ou plusieurs protéines, tel que la masse des complexes ainsi constitués soit comprise entre 50 et 100 kDa.

*Considérations sur la phosphorylation*

Ces peptides se retrouvent dans une approche ciblée utilisant le gallium comme molécule d'affinité. L'affinité des acides aminés phosphorylés pour le gallium (Posewitz MC, Tempst P., Immobilized gallium(III) *affinity chromatography* of *phosphopeptides.* Anal Chem. 1999 Jul 15; 71 (14) : 2883-92.) étant reconnu, il est donc possible d'émettre l'hypothèse que soit ces peptides, soit l'une des molécules qui leur sont associées, possèdent un ou plusieurs acides aminés phosphorylés.

Considérations *sur les* écarts de *masses* et *possible identification*

Il s'agit de considérations sur l'écart de masse entre deux des trois peptides.
PM3442 - PM3371 = 71 Da → alanine (A)
PM3486 - PM3371 = 115 Da → acide aspartique (D)
PM3486 - PM3442 = 44 Da → remplacement d'un acide aspartique (D) par une alanine (A).
Issues des banques de données, seules les séquences des défensines neutrophiles humaines *(Human Neutrophil Defensins,* ou HNPs) correspondent aux critères énoncés (voir Tableau 21).

| Tableau 21 : Séquence d'acides aminés des α-Defensines (HNP-1, HNP-2 et HNP-3) | | | |
|---|---|---|---|
| Noms | Séquence d'acides aminés | Poids Moléculaires (Da) | Différence par rapport à HNP-2 |
| Défensine-2 neutrophile humaine | -*CYCR*IPACIAGERRYGTCIYQGRLWAFCC | 3371.01 | - |
| Défensine-1 neutrophile humaine | *ACYCR*IPACIAGERRYGTCIYQGRLWAFCC | 3442.09 | *A* |
| Défensine-3 neutrophile humaine | *DCYCR*IPACIAGERRYGTCIYQGRLWAFCC | 3486.10 | *D* |

Identification des clusters PM6423, PM6439, PM6620 et PM6636 :

*Considérations sur la masse native*

Ces quatre peptides ont des propriétés biophysicochimiques corrélées. Ils sont présents dans une fraction native des échantillons telle que la masse des produits soit comprise entre 50 et 100 kDa ainsi que dans une fraction de masse native supérieure à 100 kDa. Il s'agit donc de peptides associés à plusieurs protéines distinctes, tels que la masse des complexes ainsi constitués soit comprise entre 50 et 100 kDa, ou supérieure à 100 kDa.

*Considérations sur les écarts de masses et possible identification*

Il s'agit de considération sur l'écart de masse entre deux des quatre peptides.
PM6636 - PM6620 = 16 Da → Possible hydrolyse d'une proline en hydroxyproline
PM6636 - PM6439 = 197 Da +/- 1Da → valine + proline (196 Da) ou Proline + Thréonine (198 Da)
PM6636 - PM6423 = 181 Da +/- 1Da → Pas de combinaison d'acides aminés
PM6620 - PM6439 = 181 Da +/- 1Da → Pas de combinaison d'acides aminés
PM6620 - PM6423 = 197 Da +/- 1Da → valine + proline (196 Da) ou proline + thréonine (198 Da)
PM6439 - PM6423 = 16 Da → Possible hydrolyse d'une proline en hydroxyproline
Ces considérations sont résumées dans la figure 34.

**E - CONCLUSIONS**

Les résultats relatifs à 19 marqueurs discriminants des populations Contrôle et T21 sont présentés ci-dessus. L'ensemble des marqueurs présentés au Tableau 14 peuvent être inclus dans des combinaisons, entre eux, ou en combinaison avec le triple test ou bien avec un ou deux marqueurs du triple test. Ces marqueurs, sur-exprimés ou sous-exprimés, dans le groupe contrôle ou dans le groupe malade, présentent tous un intérêt diagnostic.

En particulier, les pics de masse PM 3371, PM3442, PM 3486, identifiés comme des défensines, sont sous-exprimés dans les groupe T21 et sur-exprimés ou normalement exprimés chez les contrôles.

Les pics de masse PM 6423, PM 6439, PM6620, PM6636, sont identifiables deux à deux à une hydrolyse d'une proline en hydroxyproline (+16 Da).

**Revendications**

1. Utilisation d'un ou plusieurs marqueur(s) choisi(s) parmi : PM3061 ; PM3101 ; PM3156 ; PM3166 ; PM3181 ; PM3214 ; PM3223 ; PM3233 PM3313 ; PM3320 ; PM3321 ; PM3371 ; PM3394; PM3400; PM3442 ; PM3486 ; PM3504 ; PM3678 ; PM3705 ; PM3815 PM3896 ; PM4059 ; PM4067 ; PM4110 ; PM4111 PM4152 PM4168 ; PM4186 ; PM4197 ; PM4201 ; PM4217 PM4279; PM4286 ; PM4295 ; PM4300 ; PM4317 , PM4332 ; PM4349 ; PM4410 ; PM4412 ; PM4463 ; PM4470 ; PM4530 ; PM4583 PM4650 ; PM4682 PM4706 ; PM4722 ; PM4816 ; PM4821 PM4860 ; PM4893 ; PM4935 , PM5008 ; PM5033 ; PM5222 ; PM5625 ; PM5630 ; PM5695 ; PM5712 ; PM5720 ; PM5756; PM5793 ; PM5799 ; PM5982 ; PM6222 ; PM6299 ; PM6423 PM6430 ; PM6439 ; PM6461 ; PM6470 ; PM6482 ; PM6598 , PM6605 ; PM6620 ; PM6624 ; PM6636 ; PM6659 ;PM6679 ; PM6764 ; PM6782; PM6808 ; PM6937 ; PM7019 ; PM7020 ; PM7652 ; PM8112 ; PM8176 ; PM8206 ; PM8220 ; PM8226 ; PM8558 ; PM8563 ; PM8581 ; PM8594 ; PM8627; PM8691 ; PM8695 ; PM8717 ; PM8809 ; PM8872 ; PM8917; PM8929 ; PM9291 ; PM9358 ; PM9360 ; PM9401 ; PM9407 ; PM9617 ; PM10051 ; PM11093 ; PM11369 ; PM12434 ; PM12575 ; PM12862 ; PM13313 ; PM13316 ; PM14027 ; PM14035 ; PM14430 ; PM16672 ; PM16995,56 ; PM17262 ; PM17348,75 ; PM17386 ; PM17581,18 ; PM18901.94 ; PM19139,2 ; PM19558,27 ; PM19624,73 ; et PM19845,94, pour le diagnostic prénatal d'une anomalie chromosomique.

2. Procédé de dépistage prénatal *in vitro* d'anomalies chromosomiques, **caractérisé en ce qu'**il comprend une étape de dosage, dans un échantillon biologique provenant d'une femme enceinte, d'au moins un marqueur choisi parmi : PM3061 ; PM3101 ; PM3156 ; PM3166 ; PM3181 ; PM3214 ; PM3223 ; PM3233 ; PM3313 ; PM3320 ; PM3321 ; PM3371 ; PM3394 ; PM3400 ; PM3442 ; PM3486 ; PM3504 ; PM3678 ; PM3705 ; PM3815 ; PM3846 ; PM4059 ; PM4067 ; PM4110 ; PM4111 ; PM4152 ; PM4168 ; PM4186 ; PM4197; PM4201 ; PM4217 ; PM4279 ; PM4286 ; PM4295 ; PM4300 ; PM4317 ; PM4332 ; PM4349 ; PM4410 ; PM9912 ; PM4463 ; PM4470 ; PM4530 ; PM4583 ; PM4650 ; PM4682 ; PM4706 ; PM4722 ; PM4816 ; PM4821 ; PM4860 ; PM4893 ; PM4935 ; PM5008 ; PM5033 ; PM5222 ; PM5625 ; PM5630 ; PM5695 ; PM5712 ; PM5720 ; PM5756 ; PM5793 ; PM5799 ; PM5982 ; PM6222 ; PM6299 ; PM6423 ; PM6430 ; PM6439 ; PM6461 ; PM6470 ; PM6482 ; PM6548 PM6605 ; PM6620 ; PM6624 ; PM6636 ; PM6659 ; PM6679 ; PM6764 ; PM6782 ; PM6808; PM6937 ; PM7019 ; PM7020 ; PM7652 ; PM8112 ; PM8176 ; PM8206 ; PM8220 ; PM8226 ; PM8558 ; PM8563 ; PM8581 ; PM8594 ; PM8627 ; PM8691 ; PM8695 ; PM8717 ; PM8809 ; PM8872 ; PM8917 ; PM8929 ; PM9291 ; PM9358 ; PM9360 ; PM9401 PM9407 ; PM9617 ; PM10051 PM11093 ; PM11369 ; PM12434 ; PM12575 ; PM12862 ; PM13313 ; PM13316 ; PM14027 ; PM14035 ; PM14430 ; PM16672 ; PM16995,56 ; PM17262 ; PM17348,75 ; PM17386 ; PM17581,18 ; PM18901,94 ; PM19139,2 ; PM19558,27 ; PM19624,73 ; et PM19845,94, et d'au moins deux autres marqueurs choisis parmi cette même liste ou parmi l'hormone gonadotrophine chorionique (hCG); la sous-unité β de l'hCG; l'oestriol non conjugué (uE3); l'alpha-foetoprotéine (AFP), l'inhibitine A; et la protéine plasmatique associée à la grossesse (PAPP-A).

3. Utilisation selon la revendication 1, ou procédé selon la revendication 2, **caractérisés en ce qu'**au moins un marqueur est choisi parmi : PM3371 ; PM3442 ; PM3486 ; PM4152 ; PM4168 ; PM4186 ; PM9201 ; PM4300 ; PM5695 ; PM6222 ; PM4682 ; PM5625 , PM6423 ; PM6439 ; PM6620 ; PM6636 ; PM9360 ; PM14035 ; et PM17262.

4. Utilisation ou procédé selon l'une quelconque des revendications 1 à 3, **caractérisés en ce qu'**au moins un marqueur est sélectionné parmi PM3371, PM3442, et PM3486.

5. Utilisation ou procédé selon l'une quelconque des revendications 1 à 4, **caractérisés en ce qu'**au moins un marqueur est sélectionné parmi PM6423, PM6439, PM6620, et PM6636.

6. Utilisation ou procédé selon l'une quelconque des revendications 1 à 5, **caractérisés en ce qu'**au moins trois marqueurs sont choisis parmi : PM3061 ; PM3101 ; PM3156 ; PM3166 ; PM3181 ; PM3214; PM3223 ; PM3233 ; PM3313 ; PM3320 ; PM3321 ; PM3371 ; PM3394 ; PM3400 ; PM3442 ; PM3486 ; PM3504 ; PM3678 PM3705 ; PM3815 ; PM3846 PM4059 PM4067 ; PM4110 ; PM9111 ; PM4152 ; PF14168 ; PM4186 ; PM4197 ; PM4201 ; PM4217 ; PM9279 ; PM4286 ; PM4295 ; PM4300 ; PM4317 ; PM9332 ; PM4349 ; PM4410 ; PM4412 ; PM4463 ; PM9470 ; PM4530 ; PM4583 ; PM4650 ; PM4682 ; PM4706 ; PM4722 ; PM4816 ; PM4821 ; PM4860 ; PM4893 ; PM4935 ; PM5008 ; PM5033 ; PM5222 ; PM5625 ; PM5630 ; PM5695 ; PN5712 ; PM5720 ; PM5756 ; PM5793 ; PM5799 ; PM5982 ; PM6222 ; PM6299 ; PM6423 ; PM6430 ; PM6439 ; PM6461 ; PM6470 ; PM6482 ; PM6548 ; PM6605 ; PM6620 ; PM6624 ; PM6636 ; PM6659 ; PM6679 ; PM6764 ; PM6782 ; PM6808 , PM6937 PM7019 ; PM7020 ; PM7652 ; PM8112 ; PM8176 ; PM8206 ; PM8220 ; PM8226 ; PM8558 ; PM8563 ; PM8581 ; PM8594 ; PM8627 ; PM8691 ; PM8695 ; PM8717; PM8809 ; PM8872 ; PM8917 ; PM8929 ; PM9291 ; PM9358 ; PM9360 ; PM9401 ; PM9407 ; PM9617 ; PM10051 ; PM11093 ; PM11369 ; PM12434 ; PM12575 ; PM12862 ; PM13313 ;

PM13316 ; PM19027 , PM14035 ; PM14930 ; PM16672 ; PM16995,56 ; PM17262 ; PM17348,75 ; PM17386 ; PM17581,18 ; PM18901,94 ; PM19139,2 ; PM19558,27 ; PM19624,73 ; et PM19845,94.

7. Procédé selon l'une quelconque des revendications 2 à 6, **caractérisé en ce qu'**il comporte également, pour chaque marqueur dosé, une étape de comparaison de la concentration mesurée de ce marqueur dans l'échantillon biologique de la femme enceinte, à des valeurs de référence de la concentration de ce marqueur chez des femmes enceintes portant des foetus normaux, et chez des femmes enceintes portant des foetus atteints d'une anomalie chromosomique et/ou génétique connue, la comparaison étant indicatrice du risque que la femme enceinte porte un foetus atteint d'une anomalie chromosomique et/ou génétique.

8. Utilisation ou procédé selon l'une quelconque des revendications 1 à 7, **caractérisés en ce que** les anomalies chromosomiques dépistées sont sélectionnées dans le groupe constitué par le syndrome de Down (trisomie 21), le syndrome d'Edwards (trisomie 18), le syndrome de Patau (trisomie 13), le syndrome de Turner, le syndrome de Klinefelter, le syndrome de 1'X fragile, et le syndrome penta X.

9. Utilisation ou procédé selon la revendication 8, **caractérisés en ce qu'**une anomalie chromosomique faisant l'objet du dépistage est le syndrome de Down.

10. Procédé selon l'une quelconque des revendications 2 à 9, **caractérisé en ce qu'**il comporte une étape initiale de préparation de l'échantillon.

11. Procédé selon la revendication 10, **caractérisé en ce que** l'étape initiale de préparation de l'échantillon comprend un fractionnement en fonction de la masse des protéines.

12. Procédé selon la revendication 10 ou 11, **caractérisé en ce que** l'étape initiale de préparation de l'échantillon comprend une étape de déplétion en albumine ou autres protéines de transport.

13. Procédé selon l'une quelconque des revendications 10 à 12, **caractérisé en ce que** l'étape initiale de préparation de l'échantillon comprend une étape de purification des protéines sur une surface préparative de type anionique et/ou hydrophobe.

14. Procédé selon l'une quelconque des revendications 2 à 13, **caractérisé en ce que** le dosage d'au moins un marqueur est effectué par spectrométrie de masse.

15. Utilisation d'une ou plusieurs défensines neutrophiles humaines choisies parmi HND-1, HND-2 et HND-3, comme marqueur(s) pour le diagnostic prénatal d'une anomalie chromosomique.

16. Trousse de diagnostic prénatal d'anomalies chromosomiques, comprenant des moyens nécessaires à la mise en oeuvre d'un procédé selon l'une quelconque des revendications 2 à 14.

17. Trousse selon la revendication 16, comprenant des anticorps et/ou des aptamères spécifiques d'un ou plusieurs marqueurs sélectionnés parmi les marqueurs cités dans l'une quelconque des revendications 1 à 5.

18. Trousse selon la revendication 16 ou la revendication 17, comprenant, pour au moins un des marqueurs cités dans la revendication 1, un jeu d'anticorps spécifiques non recouvrant.

19. Trousse selon la revendication 18, dans laquelle le jeu d'anticorps spécifiques non recouvrant comprend un anticorps de capture, immobilisé sur un support, et un anticorps de détection.

Fig. 1

Centrifugation
+
Lavage (PBS 1x pH 7,4)

Sérum   Filtration
        100 kDa

<100 kDa

Fraction > 100 kDa

Filtration
50 kDa

Centrifugation
+
Lavage (PBS 1x pH 7,4)

Fraction <50 kDa

50 kDa < Fraction < 100 kDa

## Fig. 2

**Enregistrements et Optimisation**

Conditions d'enregistrement

Laser 3

Laser 2

Laser 1

MW
en Da

3000   10000    20000    25000                    300000

Zone 1    Zone 2                    Zone 3

Zones de dépouillement

## Fig. 3

Surface Multiplexe
Physico-chimie
spécifique
Format 96, 192, 384, ...

1. Association des protéines
selon leurs propriétésphysicochimiques

Lavage

2. Elimination des protéines
non spécifiques

3. Ajout de Matrice pour le
processus LDI-TOF et profil
d'expression des protéines

Matrice

Lecture

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

T21

Contrôle

## Fig. 21

Contrôle

T21

T18

Fig. 22

Fig. 23

Fig. 24

Fig. 25

Fig. 26

MW 3371

Intensité du pic

30
20
10
0

Contrôle          T21

Nuage de points

MW 3371

Intensité du pic
(Intervalle de confiance 95%)

30
20
10
0

Contrôle          T21

Boîtes et barres d'erreur

Fig. 27

MW 3442

Intensité du pic

30
20
10
0

Contrôle          T21

Nuage de points

MW 3442

Intensité du pic
(Intervalle de confiance 95%)

30
20
10
0

Contrôle          T21

Boîtes et barres d'erreur

Fig. 28

MW 3486

Nuage de points

MW 3486

Boîtes et barres d'erreur

## Fig. 29

MW6423

Nuage de points

MW6423

Boîtes et barres d'erreur

## Fig. 30

Courbes ROC (Intervalle de confiance 95%)

Fig. 31

Fig. 32

Analyse en Composante Principale - Surface échangeuse d'anions Q10

Nombre de composantes

## Fig. 33

## Fig. 34

**Office européen**
**des brevets**

**RAPPORT PARTIEL**
**DE RECHERCHE EUROPEENNE**
qui selon la règle 45 de la Convention sur le brevet
européen est consideré, aux fins de la procédure ultérieure,
comme le rapport de la recherche européenne

Numéro de la demande

EP 04 29 2013

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 4 931 385 A (BLOCK ET AL) 5 juin 1990 (1990-06-05) * exemple 6 * | 16-19 | C12Q1/68 |
| Y | US 6 492 178 B1 (PANDIAN MURUGAN R) 10 décembre 2002 (2002-12-10) * colonne 7, ligne 53-62 * * colonne 2, ligne 5-52 * | 1-4,6-19 | |
| Y | US 2003/166913 A1 (ZYMOGENETICS, INC) 4 septembre 2003 (2003-09-04) * page 8, alinéas 75,77 * * page 5, alinéa 52 * | 1-4,6-19 | |
| Y | BECKER K., BRUETON L.A., MAINIE P., BANNON M.: "Behcet Disease in constitutional trisomy 8." J. MED. GENET, vol. 38, 2001, page S34, XP008043253 * abrégé * | 1-4,6-19 | |
| A | US 2004/063134 A1 (AMERSHAM BIOSCIENCES) 1 avril 2004 (2004-04-01) * le document en entier * | | |

DOMAINES TECHNIQUES
RECHERCHES (Int.Cl.7)

C12Q

-/--

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 7 mars 2005 | Helliot, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C08)

# EP 1 624 074 A1

**Office européen**
**des brevets**

**RAPPORT PARTIEL**
**DE RECHERCHE EUROPEENNE**

Numero de la demande

EP 04 29 2013

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 5 100 806 A (MACRI ET AL) 31 mars 1992 (1992-03-31) * le document en entier * ----- | | |
| A | PETRAGLIA F ET AL: "Serum activin A and inhibitin A levels in pregnancies complicated by IUGR" HORMONE RESEARCH (BASEL), vol. 50, no. SUPPL. 3, septembre 1998 (1998-09), page 1, XP008043311 37TH ANNUAL MEETING OF THE EUROPEAN SOCIETY FOR PAEDIATRIC ENDOCRINOLOGY; FLORENCE, ITALY; SEPTEMBER 24-27, 1998 ISSN: 0301-0163 * le document en entier * ----- | | DOMAINES TECHNIQUES RECHERCHES (Int.Cl.7) |
| A | BENN P A: "Advances in prenatal screening for Down syndrome: I. General principles and second trimester testing" CLINICA CHIMICA ACTA 2002 NETHERLANDS, vol. 323, no. 1-2, 2002, pages 1-16, XP008043309 ISSN: 0009-8981 * le document en entier * ----- | | |

EPO FORM 1503 03.82 (P04C11)

**Office européen**
**des brevets**

**RECHERCHE INCOMPLETE**
**FEUILLE SUPPLEMENTAIRE C**

Numéro de la demande

EP 04 29 2013

Revendications ayant fait l'objet de recherches complètes:
    4, 7-14

Revendications ayant fait l'objet de recherches incomplètes:
    1-3,6,15-19

Revendications n'ayant pas fait l'objet de recherches:
    5

Raison pour la limitation de la recherche:

1- Les revendication 1-3, 6 15-19, qui se réfèrent à l'utilisation de marqueurs pour le diagnostic prénatal d'une anomalie chromosomique ou au dépistage anténatal in vitro d'anomalie chromosomique utilisant au moins un des marqueurs inventoriés dans la revendication 2 et au moins deux des marqueurs choisis parmi cette même liste ou parmi hCG, beta-hCG, uE3, AFP, inhibitine A et PAPP-A ou à une trousse les contenant, manquent de clarté et/ou de concision (Art. 84 CBE) car lesdits marqueurs inventoriés dans lesdites revendications 1-3 ne sont identifiés que par leur poids moléculaire mesuré par spectrométrie de masse (p. 9, l. 10-15), ce qui ne suffit pas à l'homme du métier pour les identifier sans ambiguïté. Le manque de clarté et/ou de concision au sens de l'Article 84 CBE qui s'en suit, est d'une importance telle qu'une recherche significative de l'objet des revendications devient impossible. Par conséquent, la recherche a été effectuée pour les parties de la demande qui apparaissent être claires, c'est à dire pour les marqueurs PM 3371, 3442 et 3486, correspondant respectivement aux protéines HND-2, HND-1 et HND-3 (p. 11, l. 9-13).

2- La revendication 5, qui se réfère à l'utilisation et ou au procédé des revendications 1-4 caractérisé en ce qu'au moins un marqueur sélectionné parmi PM6423, 6439, 6620 et 6630 sont utilisé, manque de clarté au sens de l'Art. 84 CBE, car lesdits marqueurs inventoriés dans ladite revendication 5 ne sont identifiés que par leur poids moléculaire mesuré par spectrométrie de masse (p. 9, l. 10-15), ce qui ne suffit pas à l'homme du métier pour les identifier sans ambiguïté. Aucune recherche est de ce fait rendu possible.

**ANNEXE AU RAPPORT DE RECHERCHE EUROPEENNE
RELATIF A LA DEMANDE DE BREVET EUROPEEN NO.**

EP 04 29 2013

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-03-2005

| Document brevet cité au rapport de recherche | | | Date de publication | Membre(s) de la famille de brevet(s) | | | Date de publication |
|---|---|---|---|---|---|---|---|
| US 4931385 | | A | 05-06-1990 | US | 5102788 | A | 07-04-1992 |
| US 6492178 | | B1 | 10-12-2002 | US | 6127186 | A | 03-10-2000 |
| | | | | US | 2003059946 | A1 | 27-03-2003 |
| | | | | AU | 2606800 | A | 01-08-2000 |
| | | | | CA | 2358504 | A1 | 20-07-2000 |
| | | | | EP | 1141703 | A1 | 10-10-2001 |
| | | | | WO | 0042428 | A1 | 20-07-2000 |
| US 2003166913 | | A1 | 04-09-2003 | US | 6576755 | B1 | 10-06-2003 |
| | | | | US | 2003143671 | A1 | 31-07-2003 |
| | | | | US | 2003157638 | A1 | 21-08-2003 |
| | | | | US | 2003166912 | A1 | 04-09-2003 |
| | | | | AU | 744152 | B2 | 14-02-2002 |
| | | | | AU | 9391598 | A | 29-03-1999 |
| | | | | CA | 2303421 | A1 | 18-03-1999 |
| | | | | EP | 1012285 | A1 | 28-06-2000 |
| | | | | JP | 2001515720 | T | 25-09-2001 |
| | | | | WO | 9913080 | A1 | 18-03-1999 |
| US 2004063134 | | A1 | 01-04-2004 | US | 2002102252 | A1 | 01-08-2002 |
| | | | | AU | 3087801 | A | 14-08-2001 |
| | | | | AU | 3087901 | A | 14-08-2001 |
| | | | | AU | 3088001 | A | 14-08-2001 |
| | | | | AU | 3088101 | A | 14-08-2001 |
| | | | | AU | 3088201 | A | 14-08-2001 |
| | | | | AU | 3088301 | A | 14-08-2001 |
| | | | | AU | 3275701 | A | 14-08-2001 |
| | | | | AU | 3275801 | A | 20-11-2001 |
| | | | | AU | 3275901 | A | 14-08-2001 |
| | | | | AU | 3276001 | A | 14-08-2001 |
| | | | | AU | 3311401 | A | 14-08-2001 |
| | | | | AU | 3658901 | A | 14-08-2001 |
| | | | | AU | 6343201 | A | 11-12-2001 |
| | | | | EP | 1158049 | A1 | 28-11-2001 |
| | | | | EP | 1309723 | A2 | 14-05-2003 |
| | | | | EP | 1309724 | A2 | 14-05-2003 |
| | | | | EP | 1292704 | A2 | 19-03-2003 |
| | | | | EP | 1325149 | A2 | 09-07-2003 |
| | | | | EP | 1292705 | A2 | 19-03-2003 |
| | | | | EP | 1290216 | A2 | 12-03-2003 |
| | | | | EP | 1341930 | A2 | 10-09-2003 |
| | | | | EP | 1332224 | A2 | 06-08-2003 |
| | | | | EP | 1325150 | A2 | 09-07-2003 |
| | | | | EP | 1309725 | A2 | 14-05-2003 |
| | | | | EP | 1290217 | A2 | 12-03-2003 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE**
**RELATIF A LA DEMANDE DE BREVET EUROPEEN NO.**

EP 04 29 2013

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-03-2005

| Document brevet cité au rapport de recherche | Date de publication | Membre(s) de la famille de brevet(s) | Date de publication |
|---|---|---|---|
| US 2004063134 A1 | | GB 2360284 A ,B | 19-09-2001 |
| | | GB 2361238 A ,B | 17-10-2001 |
| | | GB 2373500 A ,B | 25-09-2002 |
| | | GB 2374929 A | 30-10-2002 |
| | | GB 2383043 A | 18-06-2003 |
| | | GB 2375539 A ,B | 20-11-2002 |
| | | GB 2375111 A ,B | 06-11-2002 |
| | | GB 2374872 A | 30-10-2002 |
| | | GB 2378754 A ,B | 19-02-2003 |
| | | GB 2382814 A ,B | 11-06-2003 |
| | | GB 2385053 A ,B | 13-08-2003 |
| | | GB 2376018 A | 04-12-2002 |
| | | GB 2376237 A | 11-12-2002 |
| | | GB 2380197 A | 02-04-2003 |
| | | GB 2396351 A ,B | 23-06-2004 |
| | | GB 2396352 A ,B | 23-06-2004 |
| | | GB 2397376 A ,B | 21-07-2004 |
| | | JP 2004512494 T | 22-04-2004 |
| | | JP 2004501617 T | 22-01-2004 |
| | | WO 0157270 A2 | 09-08-2001 |
| | | WO 0157271 A2 | 09-08-2001 |
| | | WO 0157272 A2 | 09-08-2001 |
| | | WO 0157273 A2 | 09-08-2001 |
| | | WO 0186003 A2 | 15-11-2001 |
| US 5100806 A | 31-03-1992 | AUCUN | |

Pour tout renseignement concernant cette annexe : voir Journal Officiel de l'Office européen des brevets, No.12/82

EPO FORM P0460

Annexe aux documents de la demande - listage des séquences déposé ultérieurement

SEQUENCE LISTING

<110>  NEUROLAB

<120>  MARQUEURS ET PROCEDES POUR LE DEPISTAGE PRENATAL D'ANOMALIES CHROMOSOMIQUES

<130>  VMACp1701/2EP

<140>  04292013.2
<141>  2004-08-06

<160>  3

<170>  PatentIn version 3.1

<210>  1
<211>  30
<212>  PRT
<213>  Homo sapiens
<220>

<221>  misc_feature
<223>  Défensine-1 neutrophile humaine

<400>  1

```
Ala Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30
```

<210>  2
<211>  29
<212>  PRT
<213>  Homo sapiens
<220>

<221>  misc_feature
<223>  Défensine-2 neutrophile humaine

<400>  2

```
Cys Tyr Cys Arg Ile Pro Ala Cys Ile Ala Gly Glu Arg Arg Tyr Gly
1               5                   10                  15

Thr Cys Ile Tyr Gln Gly Arg Leu Trp Ala Phe Cys Cys
            20                  25
```

<210>  3
<211>  30
<212>  PRT
<213>  Homo sapiens
<220>

<221>  misc_feature
<223>  Défensine-3 neutrophile humaine

<400>  3

```
Asp Cys Tyr Cys Arg Ile Pro Ala Cys Ile Ala Gly Glu Arg Arg Tyr
1               5                   10                  15

Gly Thr Cys Ile Tyr Gln Gly Arg Leu Trp Ala Phe Cys Cys
            20                  25                  30
```